# EUROPÄISCHE PATENTSCHRIFT

(11) **EP 0 642 513 B1**
(45) Veröffentlichungstag und Bekanntmachung des Hinweises auf die Patenterteilung: **09.08.2000**
(21) Anmeldenummer: 93912820.3
(22) Anmeldetag: 28.05.1993
(51) Int. Cl.: C07D 487/14, A61K 31/40

(54) **INDOLOCARBAZOL-IMIDE UND DEREN VERWENDUNG**
INDOLOCARBAZOLE IMIDES AND THEIR USE
INDOLOCARBAZOL-IMIDES ET LEUR UTILISATION

(30) Priorität: 30.05.1992 DE 4217964
(43) Veröffentlichungstag der Anmeldung: 15.03.1995
(73) Patentinhaber: KTB Tumorforschungsgesellschaft mbH, 79106 Freiburg (DE)
(72) Erfinder: KLEINSCHROTH, Jürgen, D-7819 Denzlingen (DE); SCHÄCHTELE, Christoph, D-7800 Freiburg (DE); HARTENSTEIN, Johannes, D-7801 Stegen-Wittental (DE); RUDOLPH, Claus, D-7801 Vörstetten (DE); BARTH, Hubert, D-7830 Emmendingen (DE); ARANDA, Julian, D-7801 Vörstetten (DE); BETCHE, Hans, Jürgen, D-7801 Vörstetten (DE)
(74) Vertreter: Tesch, Rudolf, Dr.
(86) Internationale Anmeldenummer: EP9301347
(87) Internationale Veröffentlichungsnummer: WO9324491

(56) Entgegenhaltungen:
- EP-A- 0 388 956
- WO-A-91/18003
- JOURNAL OF BIOLOGICAL CHEMISTRY Bd. 266, Nr. 24, 1991, BALTIMORE, MD US Seiten 15771 - 15781 F. LORIOLLE ET AL. 'The bisindolylmaleimide GF 109203X is a potent and selective inhibitor of Protein Kinase C'
- JOURNAL OF ORGANIC CHEMISTRY Bd. 54, 1989, EASTON US Seiten 824 - 836 J. BERGMAN ET AL. 'Synthesis of Indolo(2,3-a)pyrrolo(3,4-c)carbazoles by double Fischer indolizations'

## Beschreibung

Gegenstand der Erfindung sind Indolocarbazol-Imide der allgemeinen Formel I 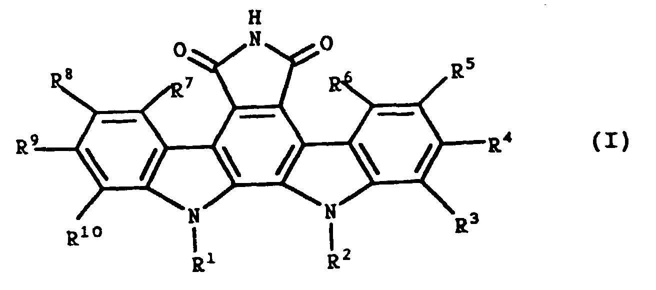 in welcher die Reste R¹ und R² gleich oder verschieden sind und Wasserstoff, Methyl, Ethyl, Cyanomethyl, 2-Cyanoethyl, 3-Cyanopropyl, 3-Cyano-2-propyl, 2-Azidoethyl, 3-Azidopropyl, 2,3-Dihydroxypropyl, 3-Chlor-2-hydroxy-1-propyl, 3-Aminopropyl, 3-Dimethylaminopropyl, 3-Trimethylammoniopropyl, 4-Aminobutyl, 5-Aminopentyl, Epoxymethyl, 2-Epoxyethyl, 3-Methylaminopropyl, 3-Ethylaminopropyl, 3-Isopropylaminopropyl, 3-Diisopropylaminopropyl, 3-Methylamino-2-hydroxy-1-propyl, 3-Ethylamino-2-hydroxy-1-propyl, 3-Isopropylamino-2-hydroxy-1-propyl, 3-Dimethylamino-2-hydroxy-1-propyl, 3-Diethylamino-2-hydroxy-1-propyl, 3-Diisopropylamino-2-hydroxy-1-propyl, 3-Pyrrolidino-2-hydroxy-1-propyl oder einen Rest -(CH₂)₂CONH₂ bedeuten und R³ bis R¹⁰ unabhängig voneinander für Wasserstoff, Methyl, Methoxy, n-Propoxy, Chlor, Brom, Nitro, Hydroxy, Amino oder zwei benachbarte Reste zusammen für Methylendioxy stehen, mit den Maßgaben, daß
(1.) nicht alle Reste R¹ bis R¹⁰ gleichzeitig Wasserstoff sein dürfen und daß
(2.) alle übrigen Reste nicht gleichzeitig Wasserstoff sein dürfen, wenn (a) R¹ eine 3-Dimethylaminopropylgruppe ist, oder wenn (b) R³ und R¹⁰ gleichzeitig Chlor, Hydroxy oder Methoxy bedeuten, oder wenn (c) R⁴ und/oder R⁹ Chlor, Hydroxy oder Methoxy bedeuten, oder wenn (d) R⁵ und/oder R⁸ Methyl, Methoxy, Benzyloxy, Chlor, Brom oder Fluor bedeuten, sowie deren pharmakologisch unbedenklichen Salze und deren Verwendung zur Herstellung von Arzneimitteln zur Behandlung und/oder Prävention von Krebs, Viruserkrankungen (z.B. HIV-Infektionen), Herz- und Gefäßerkrankungen (wie z.B. Bluthochdruck, Thrombose, Herzrhythmusstörungen, Atherosklerose), bronchopulmonalen Erkrankungen, degenerativen Erkrankungen des Zentralnervensytems (z.B. Alzheimer), Entzündungskrankheiten (z.B. Rheuma, Arthritis), Krankheiten des Immunsystems (z.B. Allergien), sowie Psoriasis oder zur Verwendung als Immunsuppressivum.

Speziell bevorzugt sind Verbindungen der allgemeinen Formel I, in der R¹ und R² die vorstehenden Bedeutungen besitzen, jedoch mindestens einer der Reste R¹ oder R² von Wasserstoff verschieden ist und R³ bis R¹⁰ die vorstehenden Bedeutungen besitzen.

Verbindungen der allgemeinen Formel I, die ein chirales Zentrum in den Resten R¹ bis R¹⁰ aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Basische Verbindungen der allgemeinen Formel I, welche ein basisches Zentrum an mindestens einem der Reste R¹ bis R¹⁰ aufweisen, werden zum Zwecke der Reinigung und aus galenischen Gründen bevorzugt in kristalline, pharmakologisch verträgliche Salze übergeführt. Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die Verbindungen können in der jeweils geeigneten Formulierung enteral oder parenteral in Dosen von 1 bis 500 mg/kg, bevorzugt 1 bis 50 mg/kg verabreicht werden.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Gegenstand der Erfindung sind weiterhin neue Indolocarbazol-Imide der allgemeinen Formel I, in denen die Substituenten R¹ bis R¹⁰ die oben genannte Bedeutung haben, mit den Maßgaben, daß
(1.) nicht alle Reste R¹ bis R¹⁰ gleichzeitig Wasserstoff sein dürfen und daß
(2.) alle übrigen Reste nicht gleichzeitig Wasserstoff sein dürfen, wenn (a) R¹ eine 3-Dimethylaminopropylgruppe ist, oder wenn (b) R³ und R¹⁰ gleichzeitig Chlor, Hydroxy oder Methoxy bedeuten, oder wenn (c) R⁴ und/oder R⁹ Chlor, Hydroxy oder Methoxy bedeuten, oder wenn (d) R⁵ und/oder R⁸ Methyl, Methoxy, Benzyloxy, Chlor, Brom oder Fluor bedeuten.

Ganz besonders bevorzugt sind Verbindungen der allgemeinen Formel I, in der R¹ und R² gleich oder verschieden sind und Wasserstoff, Methyl, Ethyl, Cyanomethyl, 2-Cyanoethyl, 3-Cyanopropyl, 3-Cyano-2-propyl, 2-Azidoethyl, 3-Azidopropyl, 2,3-Dihydroxypropyl, 3-Chlor-2-hydroxy-1-propyl, 3-Aminopropyl, 3-Dimethylaminopropyl, 3-Trimethylammoniopropyl, 4-Aminobutyl, 5-Aminopentyl, Epoxymethyl, 2-Epoxyethyl, 3-Methylaminopropyl, 3-Ethylaminopropyl, 3-Isopropylaminopropyl, 3-Diisopropyl-aminopropyl, 3-Methylamino-2-hydroxy-1-propyl, 3-Ethylamino-2-hydroxy-1-propyl, 3-Isopropylamino-2-hydroxy-1-propyl, 3-Dimethylamino-2-hydroxy-1-propyl, 3-Diethylamino-2-hydroxy-1-propyl, 3-Diisopropylamino-2-hydroxy-1-propyl, 3-Pyrrolidino-2-hydroxy-1-propyl oder einen Rest -(CH₂)₂CONH₂ bedeuten und R³ bis R¹⁰ unabhängig voneinander für Wasserstoff, Methyl, Methoxy, n-Propoxy, Chlor, Brom, Nitro, Hydroxy, Amino oder zwei benachbarte Reste zusammen für Methylendioxy stehen.

Speziell bevorzugt sind Verbindungen der allgemeinen Formel I, in der R¹ und R² die vorstehenden Bedeutungen besitzen, jedoch mindestens einer der Reste R¹ oder R² von Wasserstoff verschieden ist und R³ bis R¹⁰ die vorstehenden Bedeutungen besitzen.

Verbindungen der allgemeinen Formel I, die ein chirales Zentrum in den Resten R¹ bis R¹⁰ aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Die Verbindungen I sind je nach Substitution potente Inhibitoren der Proteinkinase C oder der Myosin Light-Chain Kinase. Dadurch sind sie geeignet zur Behandlung und/oder Prävention von Krebs, Viruserkrankungen (z.B. HIV-Infektionen), Herz- und Gefäßerkrankungen (wie z.B. Bluthochdruck, Thrombose, Herzrhythmusstörungen, Atherosklerose), bronchopulmonalen Erkrankungen, degenerativen Erkrankungen des Zentralnervensystems (z.B. Alzheimer), Entzündungskrankheiten (z.B. Rheuma, Arthritis), Krankheiten des Immunsystems (z.B. Allergien), sowie Psoriasis. Darüberhinaus können die Substanzen als Immunsuppressivum eingesetzt werden.

Die Herstellung der Verbindungen I erfolgt je nach Substitution nach einem der im folgenden beschriebenen Verfahren:
A) Durch oxidative Cyclisierung von bekannten Bisindolylmaleimiden II, bei denen R¹ bis R¹⁰ eine der oben genannten Bedeutungen besitzen und R¹³ für Wasserstoff, Methyl oder eine leicht abspaltbare Schutzgruppe Z steht (Tetrahedron 1988, 44, 2887; Angew. Chem. 1990, 92, 463; EP 328026; Tetrahedron Lett. 1990, 31, 5201; EP 397060; Deutsche Patentanmeldung P 3914764.9) oder von neuen Bisindolylmaleimiden II, die in analoger Weise zur Literatur hergestellt werden, zu Verbindungen I bzw. Verbindungen III: 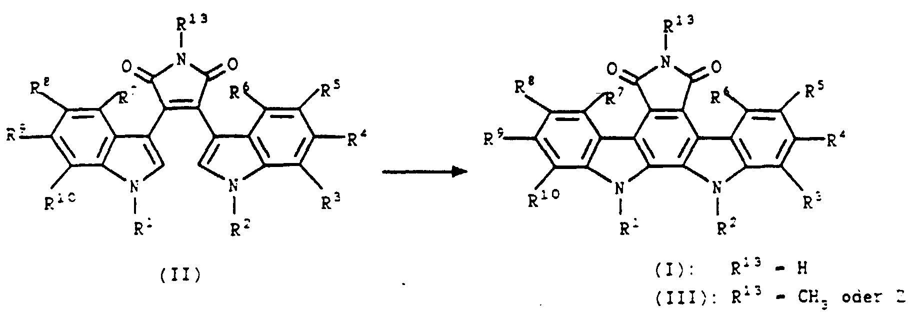 Verbindungen III, bei denen R¹ und/oder R² einen unsubstituierten, N-mono- oder N,N-disubstituierten 3-Amino-2-hydroxy-1-propylrest bedeuten, werden vorzugsweise durch Umsetzung von Verbindungen III, bei denen die entsprechenden Reste R¹ und/oder R² für einen Epoxymethyl- oder 2-Epoxyethylrest stehen, mit Ammoniak oder Aminen der allgemeinen Formel HNRR', in der R und R' für Wasserstoff oder eine C₁₋₄-Alkylgruppe stehen oder in der R und R' zusammen mit dem Stickstoffatom einen heterocyclischen Ring mit 3 bis 6 C-Atomen bilden, der auch Sauerstoff-, Schwefel und/oder weitere Stickstoffatome enthalten und durch C₁₋₄-Alkylreste substituiert sein kann, hergestellt.
   Verbindungen III, bei denen R¹³ für eine Methylgruppe steht, werden in analoger Weise zur Umsetzung von Verbindungen II, bei denen R¹³ für eine Methylgruppe steht, in Verbindungen II, bei denen R¹³ für Wasserstoff steht (Tetrahedron 1988, 44, 2887), durch Umsetzung mit Kaliumhydroxid in Wasser oder Alkoholen in die entsprechenden Anhydride übergeführt und diese durch Umsetzung mit Ammoniak, Ammoniumacetat oder mit Hexamethyldisilazan und Methanol in Dimethylformamid (Tetrahedron Lett. 1990, 5201) in an sich bekannter Weise in die entsprechenden unsubstituierten Imide I übergeführt.
   Verbindungen III, bei denen R¹³ für eine Schutzgruppe Z (z.B. SEM = 2-(Trimethylsilyl)ethoxymethylrest) steht, werden durch geeignete Abspaltung der Schutzgruppe Z (SEM z.B. mit Tetrabutylammoniumfluorid; J. Org. Chem. 1984, 49, 203) ebenfalls in die entsprechenden unsubstituierten Imide I übergeführt.
   Das beschriebene Verfahren der oxidativen Cyclisierung von Bisindolylmaleimiden II zu Indolocarbazolen I ist nicht prinzipiell neu und wurde bereits zur Synthese bekannter Indolocarbazole I angewendet (Angew. Chem. 1980, 92, 463; Tetrahedron Lett. 1985, 26, 4015; Heterocycles 1984, 21, 309; J. Org. Chem. 1987, 52, 1177; Tetrahedron 1987, 28, 4441; EP 370236). Zur Durchführung der Cyclisierung wird vorzugsweise mit einem Chinon, wie z.B. Chloranil oder 2,3-Dichlor-5,6-dicyan-p-benzochinon (DDQ) und einer geeigneten Säure, vorzugsweise p-Toluolsulfonsäure, in Toluol oder Chlorbenzol erhitzt. Die anderen in der Literatur beschriebenen Methoden für diese oxidative Cyclisierung werden in einigen Fällen ebenfalls verwendet.
B) Durch Substitution von bekannten, nach Literaturverfahren (Tetrahedron Lett. 1983, 24, 1441; J. Chem. Soc. 1990, 2475; J. Org. Chem. 1989, 54, 824; St.J. Berthel, G.W. Gribble, 197th ACS National Meeting, Dallas, Texas, 1989, Abstract 116) hergestellten, oder von neuen, in Analogie zu den Literaturverfahren, oder nach Verfahren A, hergestellten Indolo-carbazolen der allgemeinen Formeln Ia oder IIIa, 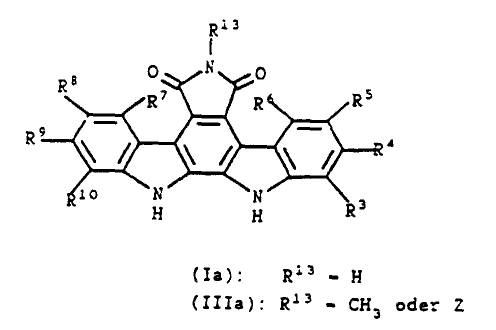 in denen R¹ und R² für Wasserstoff stehen, an einem oder an beiden Indolstickstoffatomen, z.B. durch Alkylierung mit einer Verbindung IV,

   R¹⁴-X (IV)

   in der R¹⁴ eine der für R¹ und R² angegebenen Bedeutungen besitzt und X eine geeignete Abgangsgruppe, wie z.B. Chlor, Brom, Jod oder Tosyl darstellt, unter für die Alkylierung von Indolen bzw. Carbazolen geeigneten, bekannten Reaktionsbedingungen, zu Verbindungen I oder III.

An den Indolstickstoffatomen monosubstituierte Verbindungen der allgemeinen Formeln I oder III nach Verfahren B, in denen einer der Reste R¹ oder R² für Wasserstoff steht, können durch eine weitere Substitution nach Verfahren B auch in, an den Indolstickstoffatomen disubstituierte Verbindungen der allgemeinen Formeln I oder III übergeführt werden, in denen R¹ und R² verschiedene Bedeutungen besitzen.

Verbindungen der allgemeinen Formel III nach Verfahren B werden anschließend, wie bei Verfahren A beschrieben, in Verbindungen der allgemeinen Formel I übergeführt.

Verbindungen der allgemeinen Formeln I oder III nach Verfahren B, in denen R¹ und/oder R² für einen 2-Cyanoethyl- oder 3-Cyano-2-propyl-steht, werden vorzugsweise durch basenkatalysierte Michaeladdition von Verbindungen der allgemeinen Formeln I oder III, in denen R¹ und/oder R² für Wasserstoff steht, an aktivierte Olefine der allgemeinen Formel

R¹⁵-CH=CH-R¹⁶ (V)

in der R¹⁵ für Wasserstoff oder Methyl und R¹⁶ für Cyano, oder R¹⁵ für Wasserstoff und R¹⁶ für einen Alkoxycarbonylrest mit bis zu 5 C-Atomen oder für CONH₂ steht, hergestellt. Eine besonders geeignete Base ist 1,8-Diazabicyclo[5.4]undec-7-en (DBU), als Lösungsmittel werden vorzugsweise Acetonitril oder Dimethylformamid verwendet.

Verbindungen der allgemeinen Formel I oder III nach Verfahren B, in denen R¹ und/oder R² für einen N,N-disubstituierten 3-Amino-2-hydroxy-1-propylrest steht, werden durch Alkylierung von Verbindungen der allgemeinen Formeln I oder III, in denen R¹ und/oder R² für Wasserstoff steht, mit 1,1-disubstituierten 3-Hydroxyazetidiniumhalogeniden (J. Org. Chem. 1968, 523) hergestellt.

Verbindungen der allgemeinen Formeln I oder III, in denen R¹ und R² zusammen eine Alkylengruppe mit 2 bis 4-C-Atomen bilden, werden durch Alkylierung von Verbindungen der allgemeinen Formeln Ia oder IIIa, in denen R¹ und R² für Wasserstoff stehen, mit 2 Äquivalenten einer Base, z.B. Natriumhydrid, und einem Dihalogenalkan, z.B. 1,4-Dibrombutan oder 1,3-Dibrompropan, hergestellt.

Verbindungen der allgemeinen Formel I oder III, in denen R¹ und/oder R² für einen Methyl- oder Ethylrest stehen, können auch durch Alkylierung mit Dimethyl- oder Diethylsulfat in bekannter Weise hergestellt werden.

Verbindungen der allgemeinen Formeln I und III, in denen R¹ und/oder R² für eine Cyanogruppe stehen, werden durch Umsetzung von Verbindungen der allgemeinen Formeln I oder III, in denen R¹ und/oder R² für Wasserstoff stehen, mit Natriumhydrid und Phenylcyanat in Dimethylformamid in Analogie zur Literatur (Tetrahedron Lett. 1990, 31, 3681) hergestellt.

Bei Verbindungen der allgemeinen Formeln I oder III, in denen einer oder beide der Reste R¹ und/oder R² durch Umsetzung mit einem Alkylierungsmittel der allgemeinen Formel IV nach Verfahren B eingeführt wurde, können die eingeführten Reste R¹ und/oder R² anschließend durch übliche Methoden der organischen Chemie (z.B. Houben-Weyl, Methoden der Organischen Chemie, Georg Thieme Verlag, Stuttgart, 1966) so modifiziert werden, z.B. durch Hydrolyse, Etherspaltung, Amidbildung oder Reduktion, daß sie eine andere der für R¹ und R² genannten Bedeutungen erhalten. So werden z.B. Verbindungen der allgemeinen Formeln I oder III, in denen R¹ und/oder R² für einen Carboxyalkylrest stehen, vorzugsweise durch saure oder basische Hydrolyse von Verbindungen der allgemeinen Formeln I oder III, in denen R¹ und/oder R² für einen Alkoxycarbonylalkylrest stehen, hergestellt.

Verbindungen der allgemeinen Formeln I oder III, in denen R¹ und/oder R² für einen Hydroxyalkylrest stehen, werden auch durch Hydrolyse von Verbindungen der allgemeinen Formeln I oder III, in denen R¹ und/oder R² für einen Halogenalkylrest stehen, oder durch Etherspaltung von Verbindungen der allgemeinen Formeln I oder III, in denen R¹ und/oder R² für einen Alkoxyalkylrest stehen, oder durch Umsetzung von Verbindungen der allgemeinen Formeln I oder III, in denen R¹ und/oder R² für Wasserstoff stehen, mit einem Alkylenoxid, z.B. Propylenoxid, hergestellt.

Verbindungen der allgemeinen Formeln I oder III, in denen R¹ und/oder R² für einen N-unsubstituierten Aminoalkylrest stehen, werden vorzugsweise durch katalytische Hydrierung von Verbindungen der allgemeinen Formeln I oder III, in denen R¹ und/oder R² für einen Cyanoalkyl- oder einen Azidoalkylrest stehen, hergestellt. Als Katalysator wird bei Cyanoalkylresten vorzugsweise Raney-Nickel, bei Azidoalkylresten vorzugsweise Palladium auf Aktivkohle eingesetzt.

Verbindungen der allgemeinen Formeln I oder III, in denen R¹ und/oder R² für einen Amidinoalkylrest stehen, werden auch nach bekannten Methoden aus Verbindungen der allgemeinen Formeln I oder III, in denen R¹ und/oder R² für einen Cyanoalkylrest oder einen Carboxyalkylrest stehen, hergestellt.

N-Unsubstituierte Aminoalkylgruppen R¹ und/oder R² in Verbindungen der allgemeinen Formeln I oder III können nach bekannten Methoden alkyliert oder acyliert und gegebenenfalls nach weiteren bekannten Methoden in andere funktionelle Modifikationen einer unsubstituierten Aminoalkylgruppe übergeführt werden.

Hydroxyalkylgruppen R¹ und/oder R² in Verbindungen der allgemeinen Formeln I oder III können ebenfalls nach bekannten Methoden funktionell modifiziert werden, z.B. durch Acylierung zu Acyloxyalkylgruppen, zu Halogenalkylgruppen oder durch Umsetzung mit Arylsulfonsäurechloriden zu Arylsulfonyloxyalkylgruppen.

Arylsulfonyloxyalkylgruppen und Alkylsulfonyloxyalkylgruppen R¹ und/oder R² in Verbindungen der allgemeinen Formeln I oder III können in bekannter Weise durch Umsetzung mit Kaliumcyanid in Cyanoalkylgruppen, durch Umsetzung mit Natriumazid in Azidoalkylgruppen, durch Umsetzung mit Aminen in Aminoalkylgruppen oder durch Umsetzung mit Thioharnstoff in Amidinothioalkylgruppen übergeführt werden.

Alkylthioalkylgruppen R¹ und/oder R² in Verbindungen der allgemeinen Formeln I oder III können zu Alkylsulfinylalkylgruppen oder Alkylsulfonylalkylgruppen oxidiert werden.

Weitere Modifikationen der Reste R¹ und/oder R² in Verbindungen der allgemeinen Formeln I oder III nach bekannten Methoden sind möglich.

Ebenso wie für die Reste R¹ und/oder R² in Verbindungen der allgemeinen Formeln I oder III beschrieben, können auch die Reste R³ bis R¹⁰ in Verbindungen der allgemeinen Formeln I oder III, falls gewünscht, funktionell modifiziert werden.

So können z.B. Reste R³ bis R¹⁰, die für Nitro stehen, durch bekannte Methoden zu Resten R³ bis R¹⁰, die für Amino stehen, reduziert werden.

Ebenso können Reste R³ bis R¹⁰, die für Methoxy stehen, durch Etherspaltung, z.B. mit Bortribromid oder Pyridiniumhydrochlorid, oder Reste R³ bis R¹⁰, die für Benzyloxy stehen, durch katalytische Hydrierung in Reste R³ bis R¹⁰, die für Hydroxy stehen, übergeführt werden.

Reste R³ bis R¹⁰, die für Hydroxy stehen, können anschließend erneut alkyliert, acyliert oder aminoalkyliert werden.

Ebenso können Reste R³ bis R¹⁰, die für Amino stehen, alkyliert oder acyliert werden.

Reste R³ bis R¹⁰, die für Alkylthio stehen, können in bekannter Weise zu Resten R³ bis R¹⁰, die für Alkylsulfinyl oder Alkylsulfonyl stehen, oxidiert werden.

Verbindungen der allgemeinen Formeln I oder III, in der einer oder zwei der Reste R³ bis R¹⁰ für C₁₋₄-Alkyl, C₁₋₄-Acyl, Chlor, Brom oder Nitro stehen, können auch nach bekannten Methoden der elektrophilen aromatischen Substitution aus Verbindungen der allgemeinen Formeln I oder III, in der die betreffenden Reste R³ bis R¹⁰ für Wasserstoff stehen, hergestellt werden.

Stand der Technik:
1) Es sind Imide der allgemeinen Formel I bekannt, in welcher die Reste R¹ und R² für Wasserstoff stehen (EP 269025, Verbindungen der Formel IV ; J. Org. Chem. 1989, 54, 824; Angew. Chem. 1980, 92, 463; Tetrahedron Lett. 1983, 1441; St. J. Berthel, G.W. Gribble, 197th ACS National Meeting, Dallas, Texas, 1989, abstract 116). Für diese Verbindungen sind bisher keine pharmakologischen oder Proteinkinaseinhibierenden Wirkungen beschrieben.
2) Weiterhin bekannt sind Indolocarbazol-N- oder N,N'-glykoside mikrobiellen oder semisynthetischen Ursprungs, die vorwiegend mit Antitumorwirkung beschrieben wurden und deren Aglykon von der allgemeinen Formel I umfaßt wird. Aus diesen Publikationen ist jedoch nicht ableitbar, daß das Aglykon dieser Indolocarbazolglykoside bzw. die in der vorliegenden Anmeldung beschriebenen synthetischen Derivate ebenfalls pharmakologische Wirkungen besitzen (z.B. U.S. 4,552,842; U.S. 4,524,145; EP 269025; EP 388962: EP 450327; EF 445736; JP 024571 und WO 8907-105-A; J6 3295-589-A; WO 8807-045-A). Eine diesen Indolocarbazolglykosiden verwandte Einzelverbindung synthetischen Ursprungs, für die ebenfalls pharmakologische Wirkungen angegeben sind, ist in EP 410389 beschrieben.
3) Bei dem derzeitigen Stand der Erkenntnis muß es als überraschend gelten, daß die erfindungsgemäßen Verbindungen der allgemeinen Formel I potente Inhibitoren von Proteinkinasen, insbesondere von Proteinkinase C und/oder Myosin-Light-Chain-Kinase sind. Aus der Literatur (Bio/Technology 1990, 8, 732-5) ist bekannt, daß N-glykosidische Indolocarbazolimide wie Rebeccamycin oder AT 2433 zum Unterschied von Staurosporin Proteinkinasen nicht hemmen. Die Autoren schließen daraus, daß die Struktur des Zuckerrestes für die inhibitorische Aktivität gegenüber Proteinkinasen entscheidend ist (l.c., p.734. 1. Absatz). Bei diesem Vorurteil der Fachwelt hätte der Fachmann also annehmen müssen, daß nur N-glykosidische Indolocarbazole vom Typ des Staurosporins einen geeigneten Ausgangspunkt für die Entwicklung potenter und selektiver Proteinkinase-Inhibitoren darstellen würden. Die synthetische Erschließung solcher Derivate scheitert allerdings an den fehlenden Methoden zur Verknüpfung des Indolocarbazolsystems mit dem Glykosidrest. Eine Totalsynthese des Staurosporins ist noch nicht bewerkstelligt worden! Nicht-glykosidische Indolocarbazolimide hätten den technischen Vorteil, daß sie synthetisch erheblich leichter zugänglich sind. Es hat zwar nicht an Versuchen gefehlt, nicht-glykosidische Indolocarbazolimide als Inhibitoren von Proteinkinasen zu entwickeln. In der Literatur wurde jedoch darauf hingewiesen (J. Biol. Chem. 1991, 266, 15771-15781), daß die oxidative Cyclisierung von Bisindolylmaleimiden zu Indolocarbazolimiden mit einer Abnahme der inhibitorischen Aktivität und einem "vollständigen Verlust der Spezifität" einhergeht. Daß die erfindungsgemäßen Verbindungen der allgemeinen Formel I sich als potente und, teilweise, selektive Inhibitoren von PKC und MLCK erwiesen, war also bei diesem Sachverhalt nicht zu erwarten. Im übrigen haben wir in unseren Testsystemen für die Verbindung 16 der oben genannten Literatur (J. Biol. Chem., l.c.), Beispiel 7a in unserer Beschreibung, überraschenderweise gefunden, daß es sich um einen potenten MLC-Kinase-Inhibitor handelt, der daher für die von uns beanspruchten potentiellen Arzneimittelwirkungen in Frage kommt.
4) In EP 388956 ist zwar ein mikrobiell produziertes nichtglykosidisches Indolocarbazolimid (BE-13793C) mit Antitumor-Wirkung beschrieben (Formel I, R³ = R¹⁰ = OH, R¹ - R² = R⁴ - R⁹ = H). Eine inhibitorische Aktivität gegenüber Proteinkinasen ist nicht bekannt. Nach dem Stand der Technik war anzunehmen, daß die Antitumor-Wirkung wie im Fall von Rebeccamycin und ähnlichen Verbindungen über einen anderen Mechanismus als via Hemmung von Proteinkinasen zustande kommt. Wegen der beanspruchten Antitumorwirkung für die Verbindungen der allgemeinen Formel I wird diese Verbindung aus dem allgemeinen Claim ausgeschlossen. Weiterhin sind N-Glykoside von BE-13793C mit Antitumorwirkung bekannt (WO 9118003).
5) Krebs ist eine multifaktorelle Fehlregulation von Zellwachstum. Die Aktivierung von PKC bzw. die Überexpression dieses Enzyms ist ursächlich für bestimmte Tumoren verantwortlich gemacht worden. Zum Zwecke einer spezifischen Therapie solcher Tumoren besteht ein Bedarf an potenten und selektiven Proteinkinase C-Inhibitoren. Insofern stellen die erfindungsgemäßen Verbindungen einen Fortschritt gegenüber den bekannten Indolocarbazolderivaten mit unspezifischer Antitumor-Wirkung dar.
6) Einige Indolocarbazol-Derivate der allgemeinen Formel I sind in den Patentanmeldungen EP 328000 und EP 370236 als zwischenprodukte für die erfindungsgemäßen Verbindungen beschrieben, jedoch ohne Angaben zu pharmakologischen Wirkungen oder Proteinkinase-inhibierenden Eigenschaften.

Biologische Daten von Verbindungen der allgemeinen Formel I:
1. Hemmung des Enzyms MLC-Kinase (EC 3.6.1.3; Kinase der leichten Myosinkette; Myosin Light-Chain Kinase) Die MLC-Kinase ist ein wichtiges Schlüsselenzym für die Regulation des Glattmuskeltonus (siehe z.B. Adelstein, R.S., Eisenberg, E., Ann. Rev. Biochem. 49, 921-956, 1980). Da eine Aktivierung des Enzyms zu einer Kontraktion des glatten Muskels führt, kann erwartet werden, daß Hemmstoffe der MLC-Kinase die Glattmuskelkontraktion abschwächen und in vivo zu einer Senkung des Blutdruckes führen. In den letzten Jahren wurden bereits einige Hemmstoffe der MLC-Kinase in der Literatur beschrieben (Nakanishi, S. et al., J. Biol. Chem. 263, 6215-6219, 1988 und Mol. Pharmacol. 37, 482-488, 1990), die eindeutige antihypertensive Wirkung wurde jedoch bisher nicht nachgewiesen.
   Die Hemmung der MLC-Kinase wurde in einem in vitro Enzymtest gemessen. Zu diesem Zwecke wurde die MLC-Kinase in Anlehnung an Ngai et al. (Biochem. J. 218, 863-870, 1984) aus Hühnermägen gereinigt. Die leichte Myosinkette wurde ebenfalls aus Hühnermägen gewonnen (Hathaway, D. R., Haeberle, J. R., Anal. Biochem. 135, 37-43, 1983).
   Die Enzymaktivität wurde über den Einbau von ³²P-markiertem Phosphat unter folgender. Bedingungen bestimmt: Der Reaktionsansatz von 200 µl enthielt 50 mM MOPS-NaOH, pH 7.2, 5 mM MgCl₂, 100 µM CaCl₂, 100 nM Calmodulin, 1 mM DTT, 250 µM ATP, sowie 20 µM Myosin Light-Chain. Die Reaktion wurde durch Zusatz von 1nM MLC-Kinase gestartet. Nach 20 min Inkubation bei 30°C wurde die Reaktion mit TCA gestoppt und die Proben dann abfiltriert. Der Phosphateinbau erfolgte mittels Cerenkov-Zählung der Filter.
   Tabelle 1 zeigt die Ergebnisse aus diesem Test für eine Auswahl von Beispielen.
   Um festzustellen, ob es sich um eine selektive Hemmung der MLC-Kinase handelt, wurden die Substanzen auch in anderen Enzymtests für Proteinkinasen, u.a. für Proteinkinase C, untersucht. Die Ergebnisse aus dem PKC-Test sind ebenfalls in Tabelle 1 angegeben. Die Tabelle enthält außerdem Selektivitätsfaktoren, die durch die Bestimmung der Verhältnisse der IC₅₀-Werte berechnet wurden. Aus diesen Daten geht hervor, daß vor allem die Beispiele 4, 4a, 4b, 5, 7b, 7c, 11c und 11d als potente und selektive Hemmstoffe der MLC-Kinase betrachtet werden können, während z.B. Beispiel 1 ein potenter und selektiver Proteinkinase C-Inhibitor ist.
   Für die Beispiele 5, 7c und 11d konnte neben Potenz und Selektivität in den Kinasetests in vivo eine dosisabhängige antihypertensive Wirkung nachgewiesen werden. Als Testmodell wurde die narkotisierte, spontan hypertensive Ratte (SHR) eingesetzt. Mach intravenöser Applikation der Hemmstoffe der MLC-Kinase wurde jeweils 60 Minuten nach der Substanzapplikation die in der Tabelle 2 aufgeführte Reduktion des mittleren arteriellen Blutdruckes gefunden. Eine Beeinflußung der Herzfrequenz war kaum zu beobachten (vergl. Tab. 2).

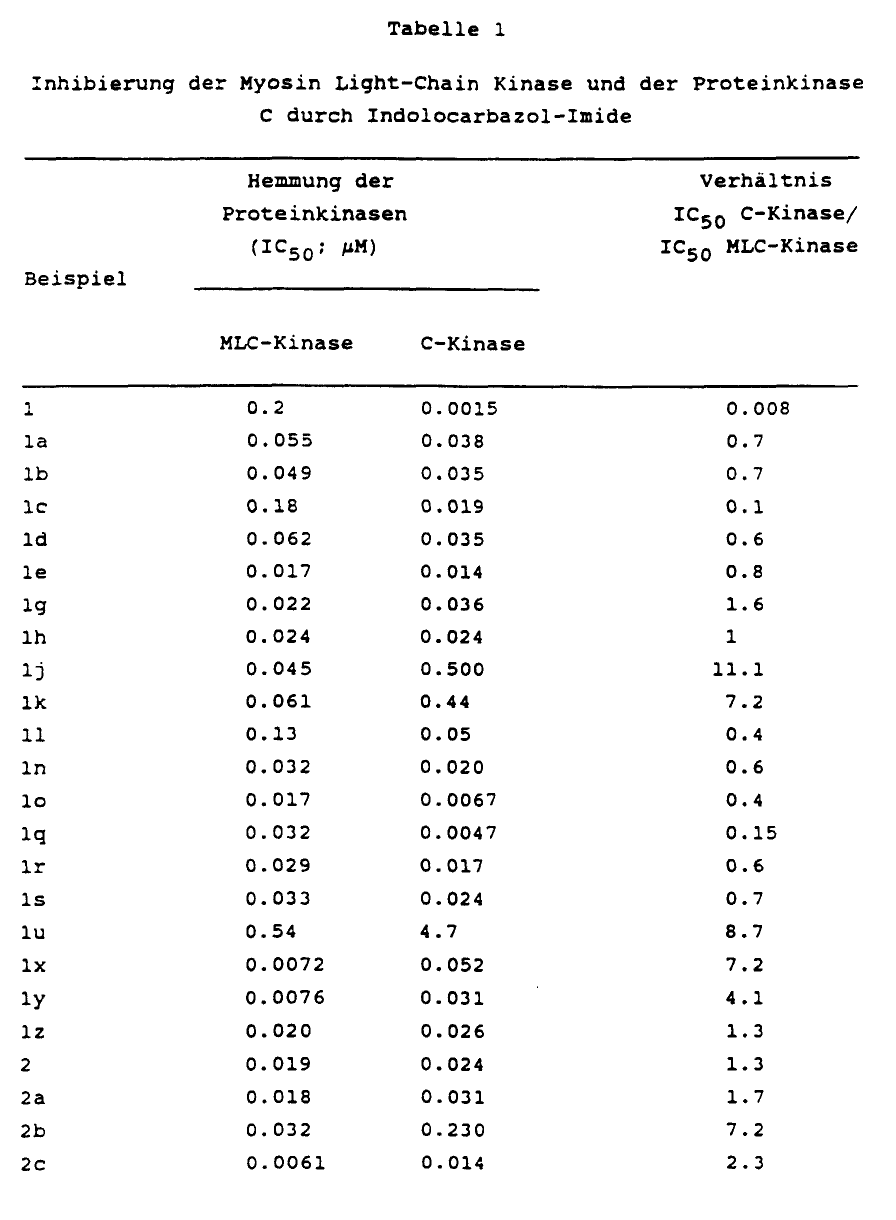

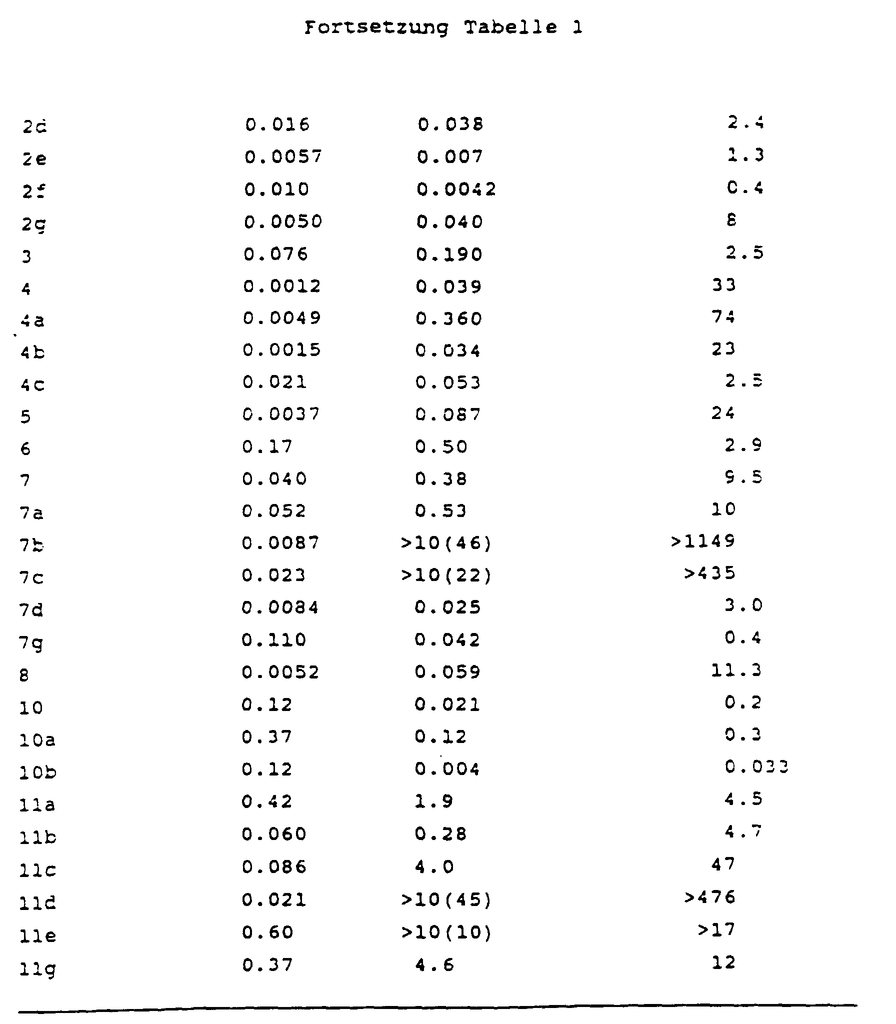

**Tabelle 2**

| Antihypertensive Wirkung von Hemmstoffen der MLC-Kinase Modell: Narkotisierte SHRs, IV-Applikation (Mittelwerte ± SD) | | | | |
|---|---|---|---|---|
| Beispiel | Dosis (mg/kg) | Reduktion des mittleren arteriellen Blutdruckes (%) | Abnahme der Herzfrequenz (%) | Zahl der Exp. |
| 5 | 1 | 15 ± 5 | 3 ± 3 | 6 |
| | 3 | 23 ± 3 | 7 ± 4 | 5 |
| | 10 | 40 ± 8 | 4 ± 3 | 6 |
| 7c | 3 | 10 ± 7 | 7 ± 3 | 6 |
| | 10 | 23 ± 2 | 8 ± 2 | 6 |
| 11d | 3 | 16 ± 3 | 6 ± 2 | 6 |
| | 10 | 35 ± 5 | 10 ± 3 | 6 |

2. In vitro Antitumor-Wirkung
Das Enzym Proteinkinase C spielt eine wichtige Rolle bei der Regulation von Zellwachstum und Differenzierung. Aufgrund dieser Eigenschaft wird die Hemmung der PKC als neues therapeutisches Prinzip in der Tumortherapie betrachtet (Gescher, A., Dale, I. L., Anti-Cancer Drug Design 4, 93-105, 1989; Grunicke, H. et al., Adv. Enzyme Regulation 28, 201-216, 1989).
Eine Möglichkeit in vitro die Antitumor-Wirkung von Substanzen zu testen, stellt der Kolonie-Test dar (Fiebig, H. H. et al., Eur. J. Canc. Clin. Onc. 23, 937-948, 1987). Für diesen Test werden menschliche Tumorzellen durch Passage auf der Nacktmaus als solider Tumor gehalten. Für den Test wird eine Einzelzellsuspension hergestellt und die Zellen dann in Schalen mit Weichagar ausplatiert. Unter diesen Bedingungen vermehren sich die Tumorzellen und bilden Kolonien. Substanzen mit Antitumor-Wirkung, wie z.B. Adriamycin, sind in der Lage, diese Koloniebildung zu verhindern.
Für die Beispiele 2c, 7g und 10 konnte gezeigt werden, daß bei bestimmten Tumortypen, das Wachstum der Tumorzellen konzentrationsabhängig gehemmt werden kann. Tabelle 3 zeigt diesen Befund exemplarisch für das Beispiel 2c.

**Tabelle 3**

| Hemmung der Koloniebildung von menschlichen Tumorzellen durch Beispiel 2c | | | | |
|---|---|---|---|---|
| Tumor-Typ | Hemmung der Koloniebildung (%) bei einer Substanzkonzentration (M) von | | | |
| | 10⁻⁷ | 10⁻⁶ | 10⁻⁵ | IC₅₀ (µM) |
| Colon | 8 | 37 | 94 | 1.7 |
| Colorectal 1 | 0 | 8 | 62 | 6.0 |
| Gastric 1 | 29 | 47 | 99 | 1.2 |
| Gastric 2 | 65 | 66 | 77 | <0.1 |
| Lung (Large Cell) | 97 | 100 | 100 | <0.1 |
| Lung (Small Cell 1) | 82 | 97 | 97 | <0.1 |
| Melanoma 1 | 48 | 81 | 89 | 0.12 |
| Melanoma 2 | 90 | 99 | 97 | <0.1 |
| Melanoma 3 | 38 | 76 | 73 | 0.21 |
| Ovarian 1 | 40 | 75 | 96 | 0.2 |
| Ovarian 2 | 5 | 33 | 75 | 2.6 |
| Ovarian 3 | 74 | 94 | 99 | <0.1 |
| Pancreas 1 | 38 | 44 | 83 | 1.5 |
| Prostata 1 | 29 | 99 | 98 | 0.2 |

3. Anti-HIV-Wirkung
Nach neueren Befunden ist die Proteinkinase C bei der Reaktivierung von HIV-Viren aus dem Latenzstadium beteiligt (Kinter, A. L. et al., J. Virol. 64, 4306-4312, 1990).
Bei der Etablierung dieser Hypothese wurde die promonozytische Zellinie U1 verwendet, die latent mit HIV-1 infiziert ist. Ohne Stimulierung kann in diesen Zellen keine Bildung von HIV gemessen werden, nach 15-minütiger Stimulierung mit dem Phorbolester PMA kommt es jedoch zu einer massiven Produktion von HIV. Wird die Zelle eine Stunde vor der PMA-Stimulierung mit dem PKC-Hemmstoff H-7 inkubiert, wobei nach der Stimulierung eine weitere 48-stündige Inkubation mit dem Hemmstoff erfolgt, so zeigt sich, daß die Produktion von HIV vollständig unterdruckt werden kann.
In diesem Modell wurden auch einige PKC-Hemmstoffe der vorliegenden Anmeldung untersucht. Als Maß für die HIV-Produktion wurde entweder mit Hilfe eines ELISA-Tests das virale Protein p24 bestimmt oder mit Hilfe einer Indikator-Zelle die HIV-vermittelte Bildung von Syncytien (= aus der Verschmelzung von Einzelzellen hervorgegangene Riesenzellen) gemessen.
Für die Beispiele 1 und 2c konnte mit beiden Testmethoden anti-HIV-Wirkung nachgewiesen werden. Die Ergebnisse sind in Tabelle 4 zusammengefaßt. Aufgrund von Kontrollexperimenten konnte nachgewiesen werden, daß die anti-HIV-Wirkung nicht durch eine cytotoxische Wirkung zustande kam.

**Tabelle 4**

| Hemmung der Phorbolester-vermittelten HIV-Reaktivierung durch PKC-Hemmstoffe | | |
|---|---|---|
| Beispiel | Hemmung der HIV-Produktion IC₅₀ (nM) | |
| | p24-Expression (Mittelwert ± SD; n=3) | Syncytien-Bildung (Mittelw. ; n=2) |
| 1 | 118 ± 98 | 28 |
| 2c | 36 ± 15 | 29 |

Die folgenden Beispiele dienen der näheren Erlauterung der Erfindung:

### Beispiel 1

### 13-(2-Cyanoethyl)-6,7,12,13-tetrahydro-3-methoxy-12-methyl-5,7-dioxo-5H-indolo[2,3-a]pyrrolo[3,4-c]carbazol

500 mg (1.18 mmol) 3-[1-(2-Cyanoethyl)-5-methoxy-3-indolyl]-4-(1-methyl-3-indolyl)-1H-pyrrol-2,5-dion in 50 ml Toluol werden mit 225 mg (1,18 mmol) p-Toluolsulfonsäure Hydrat und 315 mg (1,39 mmol) 2,3-Dichlor-5,6-dicyan-p-benzochinon (DDQ) 30 Minuten unter Rückfluß erhitzt. Nach dem Abkühlen wird im Vakuum eingedampft und der Rückstand mit 100 ml 0,1 N Natriumhydroxidlösung verrührt. Die wässrige Suspension wird mit Kochsalz gesättigt und zweimal mit je 100 ml Tetrahydrofuran extrahiert. Die Tetrahydrofuranlösungen werden zweimal mit je 100 ml gesättigter Kochsalzlösung gewaschen, über Natriumsulfat getrocknet, filtriert und eingedampft. Der Rückstand wird an Kieselgel mit Toluol/Aceton 4:1 chromatographiert. Die Fraktion mit dem Rf 0.3 wird isoliert, mit Diisopropylether verrührt und die gebildeten Kristalle abfiltriert. Man erhält 270 mg (54%) 13-(2-Cyanoethyl)-6,7,12,13-tetrahydro-3-methoxy-12-methyl-5,7-dioxo-5H- indolo[2,3-a]pyrrolo[3,4-c]carbazol in Form gelber Kristalle, die sich ab ca. 260°C zersetzen (Gasentwicklung).
Das als Ausgangsprodukt verwendete 3-[1-(2-Cyanoethyl)-5-methoxy-3-indolyl]-4-(1-methyl-3-indolyl)-1H-pyrrol-2,5-dion (dunkelgelbes amorphes Pulver, das sich ab ca. 200°C zersetzt) wird in Analogie zu EP 328 026 aus 1-(2-Cyanoethyl)-5-methoxyindol und 1-Methyl-3-indolylessigsäure hergestellt.
1-(2-Cyanoethyl)-5-methoxy-indol wird in quantitativer Ausbeute durch basenkatalysierte (DBU) Addition von Acrylnitril an 5-Methoxyindol in Acetonitril hergestellt.

In analoger Weise werden die folgenden Verbindungen erhalten:
1a.) 12-(2-Cyanoethyl)-6,7,12,13-tetrahydro-13-methyl-5,7-dioxo-5H-indolo[2,3-a]pyrrolo[3,4-c]carbazol ; Gelbe Kristalle, die sich ab ca. 200°C zersetzen.
1b.) 12-(2-Cyanoethyl)-6,7,12,13-tetrahydro-3-methoxy-13-methyl-5,7-dioxo-5H-indolo[2,3-a]pyrrolo[3,4-c]carbazol; Gelbe Kristalle, die sich ab ca. 300°C zersetzen. Das als Ausgangsprodukt verwendete 3-[1-(2-Cyanoethyl)-3-indolyl]-4-(5-methoxy-1-methyl-3-indolyl)-1H-pyrrol-2,5-dion (blaßrotes amorphes Pulver, Zers.punkt ca. 250-258°C) wird in Analogie zu EP 328026 aus 1-(2-Cyanoethyl)indol und 5-Methoxy-1-methyl-3-indolylessigsäure hergestellt.
1c.) 12-(2-Cyanoethyl)-6,7,12,13-tetrahydro-3,9-dimethoxy-13-methyl-5,7-dioxo-5H-indolo[2,3-a]pyrrolo[3,4-c]carbazol ; Gelbe Kristalle, die sich ab ca. 270°C zersetzen. Das als Ausgangsprodukt verwendete 3-[1-(2-Cyanoethyl)-5-methoxy-3-indolyl]-4-(5-methoxy-1-methyl-3-indolyl)-1H-pyrrol-2,5-dion (orangerotes amorphes Pulver, Zers.punkt ca. 255-263°C) wird in Analogie zu EP 328026 aus 1-(2-Cyanoethyl)-5-methoxy-indol und 5-Methoxy-1-methyl-3-indolylessigsäure hergestellt.
1d.) 12-(3-Cyano-2-propyl)-6,7,12,13-tetrahydro-13-methyl-5,7-dioxo-5H-indolo[2,3-a]pyrrolo[3,4-c]carbazol ; Gelbe Kristalle, die sich ab ca. 270°C zersetzen. Das als Ausgangsprodukt verwendete 3-[1-(3-Cyano-2-propyl)-3-indolyl]-4-(1-methyl-3-indolyl)-1H-pyrrol-2,5-dion (rotes amorphes Pulver, Zers.punkt ca. 218-226°C) wird in Analogie zu EP 328026 aus 1-(3-Cyano-2-propyl)indol und 1-Methyl-3-indolylessigsäure hergestellt. 1-(3-Cyano-2-propyl)indol wird in geringer Ausbeute durch basenkatalysierte (DBU) Addition von Crotonitril an Indol in Acetonitril hergestellt.
1e.) 12-Cyanomethyl-6,7,12,13-tetrahydro-13-methyl-5,7-dioxo-5H-indolo[2,3-a]pyrrolo[3,4-c]carbazol; Gelbes amorphes Pulver, das sich ab ca. 330°C zersetzt. Das als Ausgangsprodukt verwendete 3-(1-Cyanomethyl-3-indolyl)-4-(1-methyl-3-indolyl)-1H-pyrrol-2,5-dion (rotes amorphes Pulver) wird in Analogie zu EP 328026 aus 1-Cyanomethylindol (J. Amer. Chem. Soc. 1975, 97, 4098) und 1-Methyl-3-indolylessigsäure hergestellt.
1f.) 3-Chlor-13-(2-cyanoethyl)-6,7,12,13-tetrahydro-12-methyl-5,7-dioxo-5H-indolo[2,3-a]pyrrolo[3,4-c]carbazol: Gelbes amorphes Pulver vom Schmp. 281-286°C. Das als Ausgangsprodukt verwendete 3-[5-Chlor-1-(2-cyanoethyl)-3-indolyl)-4-(1-methyl-3-indolyl)-1H-pyrrol-2,5-dion (orangefarbenes amorphes Pulver vom Schmp. 240-243°C) wird in Analogie zu EP 328026 aus 5-Chlor-1-(2-cyanoethyl)indol und 1-Methyl-3-indolylessigsäure hergestellt. 5-Chlor-1-(2-cyanoethyl)indol wird durch basenkatalysierte (DBU) Addition von Acrylnitril an 5-Chlorindol in Acetonitril hergestellt.
1g.) 12-(3-Cyanopropyl)-6,7,12,13-tetrahydro-13-methyl-5,7-dioxo-5H-indolo[2,3-a]pyrrolo[3,4-c]carbazol; Gelbe Kristalle vom Schmp. 259-261°C. Das als Ausgangsprodukt verwendete 3-[1-(3-Cyanopropyl)-3-indolyl]-4-(1-methyl-3-indolyl)-1H-pyrrol-2,5-dion (dunkelrotes amorphes Pulver) wird in Analogie zu EP 328026 aus 1-(3-Cyanopropyl)indol und 1-Methyl-3-indolylessigsäure hergestellt. 1-(3-Cyanopropyl)indol wird aus 1-(2-Cyanoethyl)indol hergestellt (Synthese von 1-[3-(4-Methylphenylsulfonyloxy)propyl]indol nach J. Chem. Soc. 1967, 2599 und J. Amer. Chem. Soc. 1975, 97, 4095 und Umsetzung mit Kaliumcyanid in Dimethylformamid zu 1-(3-Cyanopropyl)indol).
1h.) 12-(3-Cyanopropyl)-6,7,12,13-tetrahydro-3-methoxy-13-methyl-5,7-dioxo-5H-indolo[2,3-a]pyrrolo[3,4-c]carbazol; Gelbes amorphes Pulver, das sich bei ca. 265°C zersetzt. Das als Ausgangsprodukt verwendete 3-[1-(3-Cyanopropyl)-3-indolyl]-4-(5-methoxy-1-methyl-3-indolyl)-1H-pyrrol-2,5-dion (rotes amorphes Pulver von Zers.punkt 275-279°C) wird in Analogie zu EP 328026 aus 1-(3-Cyanopropyl)-indol und 5-Methoxy-1-methyl-3-indolylessigsäure hergestellt.
1i.) 12-[3-(4-Methylphenylsulfonyloxy)propyl]-6,7,12,13-tetrahydro-13-methyl-5,7-dioxo-5H-indolo[2,3-a]pyrrolo[3,4-c]carbazol; Gelbes amorphes Pulver, das sich ab ca. 195°C zersetzt. Das als Ausgangsprodukt verwendete 3-[1-[3-(4-Methylphenylsulfonyloxy)propyl]]-4-(1-methyl-3-indolyl)-1H-pyrrol-2,5-dion (rotes amorphes Pulver) wird in Analogie zu EP 328026 aus 1-[3-(4-Methylphenylsulfonyloxy)propyl]indol und 1-Methyl-3-indolylessigsäure hergestellt.
1j.) 12-(3-Dimethylaminopropyl)-6,7,12,13-tetrahydro-13-methyl-5,7-dioxo-5H-indolo[2,3-a]pyrrolo[3,4-c]carbazol; Gelbe Kristalle vom Schmp. 236-243°C. Hergestellt analog Beispiel 1, jedoch mit 2 Äquivalenten p-Toluolsulfonsaure Hydrat und 2 Äquivalenten DDQ. Das als Ausgangsmaterial verwendete 3-[1-(3-Dimethylaminopropyl)-3-indolyl)-4-(1-methyl-3-indolyl)-1H-pyrrol-2,5-dion wird in Analogie zu EP 328026 aus 1-(3-Dimethylaminopropyl)indol und 1-Methyl-3-indolylessigsäure hergestellt. Beispiel 1j kann auch durch Umsetzung von Beispiel 1i mit Dimethylamin in üblicher Weise hergestellt werden.
1k.) 13-(3-Dimethylaminopropyl)-6,7,12,13-tetrahydro-3-methoxy-12-methyl-5,7-dioxo-5H-indolo[2,3-a]pyrrolo[3,4-c]carbazol; Gelbe Kristalle vom Schmp. 240-246°C. Das als Ausgangsprodukt verwendete 3-[1-(3-Dimethylaminopropyl)-5-methoxy-3-indolyl]-4-(1-methyl-3-indolyl)-1H-pyrrol-2,5-dion wird in Analogie zu EP 328026 aus 1-(3-Dimethylaminopropyl)-5-methoxyindol (hergestellt analog Synthesis 1984, 29) und 1-Methyl-3-indolylessigsäure herstellt.
1l.) 12-(3-Azidopropyl)-6,7,12,13-tetrahydro-13-methyl-5,7-dioxo-5H-indolo[2,3-a]pyrrolo[3,4-c]carbazol; Gelbe Kristalle, die sich ab ca. 220°C zersetzen. Das als Ausgangsprodukt verwendete 3-[1-(3-Azidopropyl)-3-indolyl]-4-(1-methyl-3-indolyl)-1H-pyrrol-2,5-dion wird nach EP 328026 aus 1-(3-Brompropyl)indol und 1-Methyl-3-indolylessigsäure hergestellt. Ebenso läßt sich Beispiel 1l durch Umsetzung von Beispiel 1i mit Natriumazid in Dimethylformamid herstellen.
1m.) 12-(2-Azidoethyl)-6,7,12,13-tetrahydro-13-methyl-5,7-dioxo-5H-indolo[2,3-a]pyrrolo[3,4-c]carbazol; Gelbe Kristalle vom Zers.punkt 237-241°C. Das als Ausgangsprodukt verwendete 3-[1-(2-Azidoethyl)-3-indolyl]-4-(1-methyl-3-indolyl)-1H-pyrrol-2,5-dion wird in Analogie zu Beispiel 1l und EP 328026 aus 1-(2-Chlorethyl)indol und 1-Methyl-3-indolylessigsäure hergestellt.
1n.) 3-Chlor-13-(2-cyanoethyl)-6,7,12,13-tetrahydro-9-methoxy-12-methyl-5,7-dioxo-5H-indolo[2,3-a]pyrrolo[3,4-c]carbazol; Gelbes amorphes Pulver vom Schmp >300°C. Das als Ausgangsprodukt verwendete 3-[5-Chlor-1-(2-cyanoethyl)-3-indolyl)-4-(5-methoxy-1-methyl-3-indolyl)-1H-pyrrol-2,5-dion wird in Analogie zu EP 328026 aus 5-Chlor-1-(2-cyanoethyl)indol und 5-Methoxy-1-methyl-3-indolylessigsäure hergestellt.
1o.) 13-(2-Cyanoethyl)-6,7,12,13-tetrahydro-2,3-dimethoxy-12-methyl-5,7-dioxo-5H-indolo[2,3-a]pyrrolo[3,4-c]carbazol; Gelbe Kristalle vom Schmp. 293-296°C. Das als Ausgangsprodukt verwendete 3-[1-(2-Cyanoethyl)-5,6-dimethoxy-3-indolyl)-4-(1-methyl-3-indolyl)-1H-pyrrol-2,5-dion wird in Analogie zu EP 328026 aus 1-(2-Cyanoethyl)-5,6-dimethoxy-indol und 1-Methyl-3-indolylessigsäure hergestellt. 1-(2-Cyanoethyl)-5,6-dimethoxyindol wird durch basenkatalysierte (DBU) Addition von Acrylnitril an 5,6-Dimethoxyindol in Acetonitril hergestellt.
1p.) 13-(2-Cyanoethyl)-6,7,12,13-tetrahydro-12-methyl-5,7-dioxo-3-n-propoxy-5H-indolo[2,3-a]pyrrolo[3,4-c]carbazol; Gelbes amorphes Pulver vom Schmp. 255-258°C.
1q.) 13-(3-Cyanopropyl)-6,7,12,13-tetrahydro-3-methoxy-12-methyl-5,7-dioxo-5H-indolo[2,3-a]pyrrolo[3,4-c]carbazol; Gelbes amorphes Pulver, das sich bei ca. 280-285°C zersetzt. Das als Ausgangsprodukt verwendete 3-[1-(3-Cyanopropyl)-5-methoxy-3-indolyl]-4-(1-methyl-3-indolyl)-1H-pyrrol-2,5-dion wird in Analogie zu EP 328026 aus 1-(3-Cyanopropyl)-5-methoxy-indol und 1-Methyl-3-indolylessigsäure hergestellt. 1-(3-Cyanopropyl)-5-methoxyindol wird aus 1-(3-Cyanoethyl)-5-methoxyindol analog zu 1-(3-Cyanopropyl)indol (siehe Beispiel 1g) hergestellt.
1r.) 12-(3-Cyanopropyl)-6,7,12,13-tetrahydro-3,9-dimethoxy-13-methyl-5,7-dioxo-5H-indolo[2,3-a]pyrrolo[3,4-c]carbazol; Gelbes amorphes Pulver, das sich bei ca. 285-290°C zersetzt. Das als Ausgangsprodukt verwendete 3-(1-(3-Cyanopropyl)-5-methoxy-3-indolyl]-4-(5-methoxy-1-methyl-3-indolyl)-1H-pyrrol-2,5-dion wird in Analogie zu EP 328026 aus 1-(3-Cyanopropyl)-5-methoxy-indol und 5-Methoxy-1-methyl-3-indolylessigsäure hergestellt.
1s.) 13-(2-Cyanoethyl)-6,7,12,13-tetrahydro-2,3,9-trimethoxy-12-methyl-5,7-dioxo-5H-indolo[2,3-a]pyrrolo[3,4-c]carbazol; Braunes amorphes Pulver, das sich ab ca. 275°C zersetzt. Das als Ausgangsprodukt verwendete 3-(1-(2-Cyanoethyl)-5,6-dimethoxy-3-indolyl]-4-(5-methoxy-1-methyl-3-indolyl)-1H-pyrrol-2,5-dion wird in Analogie zu EP 328026 aus 1-(2-Cyanoethyl)-5,6-dimethoxyindol und 5-Methoxy-1-methyl-3-indolylessigsäure hergestellt. 1-(2-Cyanoethyl)-5,6-dimethoxy-indol wird durch basenkatalysierte (DBU) Addition von Acrylnitril an 5,6-Dimethoxyindol in Acetonitril hergestellt.
1t.) 13-(2-Cyanoethyl)-6,7,12,13-tetrahydro-1-methoxy-12-methyl-5,7-dioxo-5H-indolo[2,3-a]pyrrolo[3,4-c]carbazol; Gelbes amorphes Pulver vom Schmp. >300°C. Das als Ausgangsprodukt verwendete 3-[1-(2-Cyanoethyl)-7-methoxy-3-indolyl]-4-(1-methyl-3-indolyl)-1H-pyrrol-2,5-dion wird in Analogie zu EP 328026 aus 1-(2-Cyanoethyl)-7-methoxyindol und 1-Methyl-3-indolylessigsäure hergestellt. 1-(2-Cyanoethyl)-7-methoxy-indol wird durch basenkatalysierte (DBU) Addition von Acrylnitril an 7-Methoxyindol in Acetonitril hergestellt.
1u.) 13-(2-Cyanoethyl)-6,7,12,13-tetrahydro-2-methoxy-12-methyl-5,7-dioxo-5H-indolo[2,3-a]pyrrolo[3,4-c]carbazol; Braunes amorphes Pulver, das sich ab ca. 200°C zersetzt. Das als Ausgangsprodukt verwendete 3-[1-(2-Cyanoethyl)-6-methoxy-3-indolyl]-4-(1-methyl-3-indolyl)-1H-pyrrol-2,5-dion wird in Analogie zu EP 328026 aus 1-(2-Cyanoethyl)-6-methoxyindol und 1-Methyl-3-indolylessigsäure hergestellt. 1-(2-Cyanoethyl)-6-methoxyindol wird durch basenkatalysierte (DBU) Addition von Acrylnitril an 6-Methoxyindol in Acetonitril hergestellt.
1v.) 12-(4-Azidobutyl)-6,7,12,13-tetrahydro-13-methyl-5,7-dioxo-5H-indolo[2,3-a]pyrrolo[3,4-c]carbazol; Gelbes amorphes Pulver vom Schmp. 186-188°C. Das als Ausgangspunkt verwendete 3-[1-(4-Azidobutyl)-3-indolyl]-4-(1-methyl-3-indolyl)-1H-pyrrol-2,5-dion wird nach EP 328026 hergestellt.
1w.) 12-(5-Azidopentyl)-6,7,12,13-tetrahydro-13-methyl-5,7-dioxo-5H-indolo[2,3-a]pyrrolo[3,4-]carbazol; Gelbes amorphes Pulver vom Schmp. 165-170°C. Das als Ausgangspunkt verwendete 3-[1-(5-Azidobutyl)-3-indolyl]-4-(1-methyl-3-indolyl)-1H-pyrrol-2,5-dion wird nach EP 328026 hergestellt.
1x.) 12-(2-Dimethylaminoethyl)-6,7,12,13-tetrahydro-13-methyl-5,7-dioxo-5H-indolo[2,3-a]pyrrolo[3,4-c]carbazol; Gelbes amorphes Pulver, das sich ab ca. 260°C zersetzt. Hergestellt analog Beispiel 1j. Das als Ausgangsprodukt verwendete 3-[1-(2-Dimethylaminoethyl)-3-indolyl]-4-(1-methyl-3-indolyl)-1H-pyrrol-2,5-dion wird in Analogie zu EP 328026 aus 1-(2-Dimethylaminoethyl)indol und 1-Methyl-3-indolylessigsäure hergestellt.
1y.) 12-(3-Dimethylaminopropyl)-6,7,12,13-tetrahydro-3-methoxy-13-methyl-5,7-dioxo-5H-indolo[2,3-a]pyrrolo[3,4-c]carbazol; Gelbes amorphes Pulver vom Schmp. 221-227°C. Das als Ausgangsprodukt verwendete 3-[1-(2-Dimethylaminopropyl)-3-indolyl]-4-(5-methoxy-1-methyl-3-indolyl)-1H-pyrrol-2,5-dion wird in Analogie zu EP 328026 aus 1-(3-Dimethylaminopropyl)indol und 5-Methoxy-1-methyl-3-indolylessigsäure hergestellt.
1z.) 13-(3-Dimethylaminopropyl)-6,7,12,13-tetrahydro-2,3-dimethoxy-12-methyl-5,7-dioxo-5H-indolo[2,3-a]pyrrolo[3,4-c]carbazol; Gelbes amorphes Pulver vom Schmp. 190-196°C. Das als Ausgangsprodukt verwendete 3-[5,6-Dimethoxy-1-(3-dimethylaminopropyl)-3-indolyl]-4-(1-methyl-3-indolyl)-1H-pyrrol-2,5-dion wird in Analogie zu EP 328026 aus 5,6-Dimethoxy-1-(3-dimethylaminopropyl)indol und 1-Methyl-3-indolylessigsäure hergestellt.
1aa.) 12-Epoxymethyl-6,7,12,13-tetrahydro-13-methyl-5,7-dioxo-5H-indolo[2,3-a]pyrrolo[3,4-c]carbazol; Braunes amorphes Pulver vom Schmp. 142-148°C. Das als Ausgangsprodukt verwendete 3-[1-Epoxymethyl-3-indolyl]-4-(1-methyl-3-indolyl)-1H-pyrrol-2,5-dion wird in Analogie zu EP 328026 aus 1-Epoxymethylindol und 1-Methyl-3-indolylessigsäure hergestellt.
1ab.) (±)-12-(3-Chlor-2-hydroxy-1-propyl)-6,7,12,13-tetrahydro-13-methyl-5,7-dioxo-5H-indolo[2,3-a]pyrrolo[3,4-c]-carbazol; Gelbes amorphes Pulver vom Schmp. 238-254°C. Hergestellt aus Beispiel 1aa.) durch Umsetzung mit Chlorwasserstoff bzw. als Nebenprodukt bei der Herstellung von Beispiel 1aa.).

### Beispiel 2

### 13-(2-Cyanoethyl) -6,7,12,13-tetrahydro-3-hydroxy-12-methyl-5,7-dioxo-5H-indolo[2,3-a]pyrrolo[3,4-c]carbazol

200 mg (0.45 mmol) 13-(2-Cyanoethyl)-6,7,12,13-tetrahydro-3-methoxy-12-methyl-5,7-dioxo-5H-indolo[2,3-a]pyrrolo[3,4-c]carbazol (Beispiel 1) in 15 ml Dichlormethan werden auf -10°C gekühlt und 0.7 ml (0,7 mmol) einer 1M Lösung von Bortribrömid in Dichlormethan zugetropft. Da sich nach 3 Stunden bei -10°C keine Umsetzung zeigte (DC), werden nochmal 1.4 ml der 1M Losung von Bortribromid in Dichlormethan zugefügt und 16 Stunden bei 20°C gerührt. Es werden 100 ml Wasser und 100 ml Tetrahydrofuran zugegeben, die organische Phase abgetrennt, mit Wasser (100 ml) gewaschen und über Natriumsulfat getrocknet.
Nach Filtration und Eindampfen wird der Rückstand an Kieselgel, zu nächst mit Dichlormethan/Methanol 95:5, dann erneut mit Toluol/Tetrahydrofuran 4:1 chromatographiert. Die Fraktion mit dem Rf 0.3 in Toluol/Tetrahydrofuran 2:1 wird isoliert, mit Diisopropylether/Aceton verrührt und die gebildeten Kristalle abfiltriert.
Man erhält 13-(2-Cyanoethyl)-6,7,12,13-tetrahydro-3-hydroxy-12-methyl-5,7-dioxo-5H-indolo[2,3-a]pyrrolo[3,4-c]carbazol in Form gelber Kristalle, die sich ab ca. 315°C zersetzen.

In analoger Weise werden die folgenden Verbindungen erhalten:
2a.) 12-(2-Cyanoethyl)-6,7,12,13-tetrahydro-3-hydroxy-13-methyl-5,7-dioxo-5H-indolo[2,3-a]pyrrolo[3,4-c]carbazol; Gelbes amorphes Pulver, das sich ab ca. 320°C zersetzt (hergestellt aus Beispiel 1b).
2b.) 13-(2-Cyanoethyl)-6,7,12,13-tetrahydro-2,3-dihydroxy-12-methyl-5,7-dioxo-5H-indolo[2,3-a]pyrrolo[3,4-c]carbazol; Oranges amorphes Pulver, das sich ab ca 270°C zersetzt. (hergestellt aus Beispiel 1o)
2c.) 12-(3-Dimethylaminopropyl)-6,7,12,13-tetrahydro-3,9-dihydroxy-5,7-dioxo-5H-indolo[2,3-a]pyrrolo[3,4-c]carbazol; Dunkelgelbe Kristalle, die sich ab ca. 200°C zersetzen. (hergestellt aus Beispiel 7)
2d.) 12-(3-Dimethylaminopropyl)-6,7,12,13-tetrahydro-3(9)-hydroxy-9(3)-methoxy-5,7-dioxo-5H-indolo[2,3-a]pyrrolo[3,4-c]carbazol; Hellbraunes amorphes Pulver, das sich ab ca. 195°C zersetzt (ca. 2:1-Regioisomerengemisch, hergestellt aus Beispiel 7).
2e.) 6,7,12,13-Tetrahydro-3,9-dihydroxy-5,7-dioxo-5H-indolo[2,3-a]pyrrolo[3,4-c]carbazol; Hellbraunes amorphes Pulver, Schmp. >350°C (hergestellt aus 6,7,12,13-Tetrahydro-3,9-dimethoxy-5,7-dioxo-5H-indolo[2,3-a]pyrrolo[3,4-c]carbazol; siehe Beispiel 11.d).
2f.) 12-(2-Cyanoethyl)-6,7,12,13-tetrahydro-3,9-dihydroxy-13-methyl-5,7-dioxo-5H-indolo[2,3-a]pyrrolo[3,4-c]carbazol; Dunkelgelbes amorphes Pulver, das sich ab ca. 270°C zersetzt (hergestellt aus Beispiel 1c).
2g.) 13-(3-Dimethylaminopropyl)-6,7,12,13-tetrahydro-3-hydroxy-12-methyl-5,7-dioxo-5H-indolo[2,3-a]pyrrolo[3,4-c]carbazol; Gelbes amorphes Pulver vom Schmp. 270-273°C. (hergestellt aus Beispiel 1k)
2h.) 12-(3-Diisopropylaminopropyl)-6,7,12,13-tetrahydro-3-hydroxy-13-methyl-5,7-dioxo-5H-indolo[2,3-a]pyrrolo [3,4-c]carbazol; Gelbe Kristalle vom Schmp. 303-306°C.

### Beispiel 3

### 12-(3-Dimethylaminopropyl)-6,7,12,13-tetrahydro-13-methyl-5,7-dioxo-5H-indolo[2,3-a]pyrrolo[3,4-c]carbazol Methojodid

57.6 mg (0.136 mmol) 12-(3-Dimethylaminopropyl)-6,7,12,13-tetrahydro-13-methyl-5,7-dioxo-5H-indolo[2,3-a]pyrrolo[3,4-c]carbazol (Beispiel 1j) werden in 50 ml Ethylacetat gelost, mit 3 Tropfen Methyljodid versetzt und 16 Stunden bei 20°C gerührt. Die ausgefallenen Kristalle werden abfiltriert, mit Ethylacetat gewaschen und getrocknet.
Man erhält das Methojodid in Form gelber Kristalle, die sich ab ca. 280°C zersetzen. DC: Kieselgel, Butylacetat/Essigester/Wasser 3:2:1, Rf = 0.45

### Beispiel 4

### 12-(3-Aminopropyl)-6,7,12,13-tetrahydro-13-methyl-5,7-dioxo-5H-indolo[2,3-a]pyrrolo[3,4-c]carbazol

760 mg (1.94 mmol) 12-(2-Cyanoethyl)-6,7,12,13-tetrahydro-13-methyl-5,7-dioxo-5H-indolo[2,3-a]pyrrolo[3,4-c]carbazol (Beispiel 1a) in 100 ml Methanol werden mit 500 mg Raney-Nickel bei 60°C und 50-65 bar Druck 20 Stunden im Autoklaven hydriert.
Es wird eingedampft, der Rückstand in 100 ml Dimethylformamid aufgenommen, vom Katalysator abfiltriert und erneut im Vakuum eingedampft. Der Rückstand wird an Kieselgel mit Dichlormethan/Methanol, NH₃ gesättigt, 95:5 chromatographiert.
Die Fraktion mit dem Rf 0.1 wird isoliert, mit Ethanol verrührt und das nicht gelöste Produkt abfiltriert. Man erhält 12-(3-Aminopropyl)-6,7,12,13-tetrahydro-13-methyl-5,7-dioxo-5H-indolo[2,3-a]pyrrolo[3,4-c]carbazol als amorphes, gelbes Pulver, das sich ab ca. 250°C zersetzt.

Beispiel 4 wird auch erhalten durch katalytische Hydrierung von Beispiel 11 mit 10% Palladium auf Aktivkohle in Ethylacetat bei Normaldruck und 20°C.

In analoger Weise zu Beispiel 4 werden die folgenden Verbindungen erhalten:
4a.) 13-(3-Aminopropyl)-6,7,12,13-tetrahydro-3-methoxy-12-methyl-5,7-dioxo-5H-indolo[2,3-a]pyrrolo[3,4-c]carbazol; Dunkelgelbes amorphes Pulver, das sich ab ca. 240°C zersetzt (hergestellt aus Beispiel 1).
4b.) 13-(3-Aminopropyl)-3-chlor-6,7,12,13-tetrahydro-9-methoxy-12-methyl-5,7-dioxo-5H-indolo[2,3-a]pyrrolo[3,4-c]carbazol; Gelbes amorphes Pulver vom Schmp. 276-281°C. (hergestellt aus Beispiel 1n).
   Bei einer Hydrierung von Beispiel 1a in Methanol/Ammoniak/Tetrahydrofuran in Gegenwart von Aceton unter den Bedingungen von Beispiel 4 wird die folgende Verbindung isoliert:
4c.) 13-(3-Isopropylaminopropyl)-6,7,12,13-tetrahydro-13-methyl-5,7-dioxo-5H-indolo[2,3-a]pyrrolo[3,4-c]carbazol; Gelbes amorphes Pulver vom Schmp. 205-210°C.

### Beispiel 5

### 12-(2-Carbamoylethyl)-6,7,12,13-tetrahydro-5,7-dioxo-5H-indolo[2,3-a]pyrrolo[3,4-c]carbazol

a) 740 mg (1.86 mmol) 12-(2-Carboxyethyl-6,7,12,13-tetrahydro-5,7-dioxo-indolo[2,3-a]furano[3,4-c]carbazol werden im Autoklaven mit 100 ml ammoniakgesättigtem Ethanol 12 Stunden auf 140°C erhitzt. Nach dem Eindampfen wird der Rückstand an Kieselgel mit Toluol/Tetrahydrofuran 4:1 chromatographiert und die Fraktion mit dem Rf 0.4 isoliert.
   Man erhält 12-(2-Carbamoylethyl)-6,7,12,13-tetrahydro-5,7-dioxo-5H-indolo[2,3-a]pyrrolo[3,4-c]carbazol in Form dunkelgelber Kristalle, die sich ab 310°C zersetzen. Das als Ausgangsprodukt verwendete 12-(2-Carboxyethyl)-6,7,12,13-tetrahydro-5,7-dioxo-indolo[2,3-a]furano[3,4-c]carbazol wird wie folgt hergestellt:
   980 mg (2.39 mmol) 12-(2-Carbamoylethyl)-6,7,12,13-tetrahydro-6-methyl-5,7-dioxo-5H-indolo[2,3-a]pyrrolo[3,4-c]carbazol werden in 200 ml 15% methanolischer Kaliumhydroxidlösung 1,5 Stunden unter Rückfluß erhitzt. Nach dem Abkühlen wird abfiltriert, das Filtrat mit 2N Salzsäure angesäuert, das ausgefallene Produkt abfiltriert und mit Wasser gewaschen. Man erhält 12-(2-Carboxyethyl)-6,7,12,13-tetrahydro-5,7-dioxo-indolo[2,3-a]furano[3,4-c]carbazol als bräunliches amorphes Pulver.
   Das als Ausgangsprodukt verwendete 12-(2-Carbamoylethyl)-6,7,12,13-tetrahydro-6-methyl-5,7-dioxo-5H-indolo[2,3-a]pyrrolo[3,4-c]carbazol wird wie folgt hergestellt:
   500 mg (1.47 mmol) 6,7,12,13-Tetrahydro-6-methyl-5,7-dioxo-5H-indolo[2,3-a]pyrrolo[3,4-c]carbazol in 50 ml Acetonitril werden mit 1,05 g (14.8 mmol) Acrylsäureamid und 0.1 ml DBU 4 Tage bei 20°C gerührt. Die Suspension wird eingedampft, mit 30 ml Dichlormethan/Methanol 95:5 verrührt und die nicht gelösten Kristalle abfiltriert.
   Man erhalt 12-(2-Carbamoylethyl)-6,7,12,13-tetrahydro-6-methyl-5,7-dioxo-5H-indolo[2,3-a]pyrrolo[3,4-c]carbazol in Fort gelber Kristalle.
   Das als Ausgangsprodukt verwendete 6,7,12,13-Tetrahydro-6-methyl-5,7-dioxo-5H-indolo[2,3-a]pyrrolo[3,4-c]carbazol wird wie folgt hergestellt:
   1 g (2.93 mmol) 3,4-Bis(indol-3-yl)-N-methylmaleinsäureimid werden mit 800 mg (3.52 mmol) DDQ und 560 mg (2.94 mmol) p-Toluolsulfonsäure Hydrat in 200 ml Toluol 1 h unter Rückfluß gekocht. Das Lösungsmittel wird zu zwei Dritteln abdestilliert, der Niederschlag abfiltriart und mit 100 ml 0.1 N Natriumhydroxidlösung ausgerührt. Es wird filtriert, der Niederschlag mit Wasser gewaschen, dann in Ethanol suspendiert und erneut abfiltriert.
   Man erhält 6,7,12,13-Tetrahydro-6-methyl-5,7-dioxo-5H-indolo[2,3-a]pyrrolo[3,4-c]carbazol als gelbes amorphes Pulver vom Schmp. >350°C.
   Das als Ausgangsmaterial verwendete 3,4-Bis(indol-3-yl)-N-methylmaleinsäureimid wird nach Literatur (Tetrahedron 1988, 44, 2887) hergestellt.
b) 12-(2-Carbamoylethyl)-6,7,12,13-tetrahydro-5,7-dioxo-5H-indolo[2,3-a]pyrrolo[3,4-c]carbazol (Beispiel 5) kann auch aus 12-(2-Cyanoethyl)-6,7,12,13-tetrahydro-6-methyl-5,7-dioxo-5H-indolo[2,3-a]pyrrolo[3,4-c]carbazol durch Umsetzung mit methanolischer Kaliumhydroxidlösung und anschließender Umsetzung mit Ammoniak in Ethanol im Autoklaven, wie unter a) beschrieben, hergestellt werden. 12-(2-Cyanoethyl)-6,7,12,13-tetrahydro-6-methyl-5,7-dioxo-5H-indolo[2,3-a]pyrrolo[3,4-c]carbazol wird durch Umsetzung von 6,7,12,13-Tetrahydro-6-methyl-5,7-dioxo-5H-indolo[2,3-a]pyrrolo[3,4-c]carbazol mit Acrylnitril und DBU in Acetonitril hergestellt, in analoger Weise zu der unter a) beschriebenen Umsetzung mit Acrylsäureamid.

### Beispiel 6

### (±)-12-(3-Diethylamino-2-hydroxy-1-propyl)-6,7,12,13-tetrahydro-5,7-dioxo-5H-indolo[2,3-a]pyrrolo[3,4-c]carbazol

285 mg (0.63 mmol) (±)-12-(3-Diethylamino-2-hydroxy-1-propyl)-6,7,12,13-tetrahydro-5,7-dioxo-indolo[2,3-a]furano[3,4-c]carbazol werden in 30 ml einer gesättigten Lösung von Ammoniak in Ethanol im Autoklaven 6 stunden auf 140°C erhitzt.
Die Lösung wird eingedampft, der Rückstand in 50 ml Diisopropylether/Tetrahydrofuran 4:1 erhitzt und die Kristalle nach den Abkühlen abfiltriert. Man erhält (±)-12-(3-Diethylamino-2-hydroxy-1-propyl)-6,7,12,13-tetrahydro-5,7-dioxo-5H-indolo[2,3-a]pyrrolo[3,4-c]carbazol in Form gelber Kristalle, die sich ab 235°C zersetzen.

Das Ausgangsprodukt wird wie folgt hergestellt:
440 mg (0.94 mmol) (±)-12-(3-Diethylamino-2-hydroxy-1-propyl)-6,7,12,13-tetrahydro-6-methyl-5,7-dioxo-5H-indolo[2,3-a]pyrrolo[3,4-c]carbazol in 100 ml 10%iger methanolischer Kaliumhydroxidlösung werden 1.5 h unter Rückfluß erhitzt. Nach dem Abkühlen wird mit halbkonzentrierter Salzsäure angesäuert, filtriert und das Filtrat eingedampft. Der Rückstand wird mit 10%iger Kaliumcarbonatlösung und Ethylacetat aufgenommen, die organische Phase mit Wasser gewaschen und über Natriumsulfat getrocknet. Es wird filtriert, eingedampft, der Rückstand mit 25 ml Diisopropylether verrührt und die gebildeten Kristalle abfiltriert.
Man erhält (±)-12-(3-Diethylamino-2-hydroxy-1-propyl)-6,7,12,13-tetrahydro-5,7-dioxo-indolo[2,3-a]furano[3,4-c]carbazol als gelbe Kristalle, die sich ab ca. 200°C zersetzen.

Das Ausgangsmaterial wird wie folgt hergestellt:
1 g (2.95 mmol) 6,7,12,13-Tetrahydro-6-methyl-5,7-dioxo-5H-indolo[2,3-a]pyrrolo[3,4-c]carbazol (Vorstufe für Beispiel 5) wird in 75 ml trockenem Dimethylformamid gelöst und zu einer Suspension von 100 mg (3.3mmol) Natriumhydrid (80%ig in Paraffinöl) in 25 ml Dimethylformamid getropft (Argonatmosphäre). Nach 1 Stunde Rühren bei 20°C werden 975 mg (5.9 mmol) 1,1-Diethyl-3-hydroxy-azetidiniumchlorid zugefügt und 3 Tage bei 20°C gerührt. Es wird im Vakuum eingedampft, der Rückstand mit Wasser (150 ml) und Ethylacetat (500 ml) aufgenommen, die organische Phase abgetrennt und über Natriumsulfat getrocknet. Nach Filtration wird eingedampft und der Rückstand an Kieselgel mit Dichlormethan/Methanol 99:1 chromatographiert. Die Fraktion mit dem Rf 0.2 in Dichlormethan/Methanol 95:5 wird isoliert, mit 20 ml Diisopropylether verrührt und die Kristalle abfiltriert. Man erhält (±)-12-(3-Diethylamino-2-hydroxy-1-propyl)-6,7,12,13-tetrahydro-6-methyl-5,7-dioxo-5H-indolo[2,3-a]pyrrolo[3,4-c]carbazol als gelbe Kristalle vom Zers.punkt 220-228°C.

### Beispiel 7

### 12-(3-Dimethylaminopropyl)-6,7,12,13-tetrahydro-3,9-dimethoxy-5,7-dioxo-5H-indolo[2,3-a]pyrrolo[3,4-c]carbazol

510 mg (1.08 mmol) 12-(3-Dimethylaminopropyl)-6,7,12,13-tetrahydro-3,9-dimethoxy-5,7-dioxo-indolo[2,3-a]furano[3,4-c]carbazol werden in 40 ml einer gesättigten Lösung von Ammoniak in Ethanol im Autoklaven 24 stunden auf 140°C erhitzt.
Nach dem Abkühlen wird der ausgefallene Niederschlag abgesaugt, an Kieselgel mit Toluol/Ethanol 10:1 chromatographiert und die Fraktion mit dem Rf 0.25 in Toluol/Ethanol 5:1 isoliert. Man erhält 12-(3-Dimethylaminopropyl)-6,7,12,13-tetrahydro-3,9-dimethoxy-5,7-dioxo-5H-indolo[2,3-a]pyrrolo[3,4-c]carbazol als gelbe Kristalle, die sich ab ca. 230°C zersetzen. Diese Verbindung kann aus demselben Ausgangsmaterial auch durch Umsetzung mit Hexamethyldisilazan und Methanol in Dimethylformamid analog zum Literaturverfahren (Tetrahedron Lett. 1990, 31, 5201) hergestellt werden.

Das Ausgangsmaterial wird aus 12-(3-Dimethylaminopropyl)-6,7,12,13-tetrahydro-3,9-dimethoxy-6-methyl-5,7-dioxo-5H-indolo[2,3-a]pyrrolo[3,4-c]carbazol durch Umsetzung mit methanolischer Kaliumhydroxidlösung, in analoger Weise wie bei der entsprechenden Vorstufe von Beispiel 6 beschrieben, hergestellt. 12-(3-Dimethylaminopropyl)-6,7,12,13-tetrahydro-3,9-dimethoxy-6-methyl-5,7-dioxo-5H-indolo[2,3-a]pyrrolo[3,4-c]carbazol wird wie folgt hergestellt:
Eine Lösung von 2,0 g (5 mmol) 6,7,12,13-Tetrahydro-3,9-dimethoxy-6-methyl-5,7-dioxo-5H-indolo[2,3-a]pyrrolo[3,4-c]carbazol in 100 ml trockenem Dimethylformamid wird bei 20°C zu einer Suspension von 182 mg (6.1 mmol) Natriumhydrid (80%ig in Paraffinöl) in 5 ml Dimethylformamid getropft. Nach 1 Stunde Rühren bei 20°C werden 738 mg (6.1 mmol) 3-Dimethylaminopropylchlorid zugefügt und 20 Stunden bei 20°C gerührt. Das Lösungsmittel wird im Vakuum abdestilliert, der Rückstand mit Ethylacetat (150 ml) und Wasser (150 ml) versetzt und der in beiden Phasen unlösliche Feststoff abfiltriert. Der Feststoff wird in Tetrahydrofuran aufgenommen, die Tetrahydrofuranlösung mit gesättigter Kochsalzlösung gewaschen, über Natriumsulfat getrocknet, filtriert und eingedampft. Der Rückstand wird zusammen mit dem Rückstand aus der Ethylacetatlösung an Kieselgel mit Ethylacetat/Methanol 4:1 chromatographiert. Die Fraktion mit dem Rf 0.2 in Ethylacetat/Methanol 3:1 wird isoliert und mit Diisopropylether/Ethylacetat 9:1 verrührt. Die gebildeten Kristalle werden abfiltriert. Man erhält 12-(3-Dimethylaminopropyl)-6,7,12,13-tetrahydro-3,9-dimethoxy-6-methyl-5,7-dioxo-5H-indolo[2,3-a]pyrrolo[3,4-c]carbazol als gelbbraune Kristalle.

6,7,12,13-Tetrahydro-3,9-dimethoxy-6-methyl-5,7-dioxo-5H-indolo[2,3-a]pyrrolo[3,4-c]carbazol wird durch oxidative Cyclisierung von 3,4-Bis(5-methoxy-indol-3-yl)-N-methylmaleinsäureimid (hergestellt analog Tetrahedron 1988, 44, 2887) mit DDQ und p-Toluolsulfonsäure Hydrat in Toluol, in analoger Weise wie bei Beispiel 5 für 3,4-Bis(indol-3-yl)-N-methylmaleinsäureimid beschrieben, hergestellt.

In analoger Weise werden die folgenden Verbindungen hergestellt:
7b.) 6,7,12,13-Tetrahydro-3,9-dimethoxy-12-methyl-5,7-dioxo-5H-indolo[2,3-a]pyrrolo[3,4-c]carbazol; Gelbe Kristalle vom Schmp. >330°C. Das als Ausgangsprodukt verwendete 6,7,12,13-Tetrahydro-3,9-dimethoxy-6,12-dimethyl-5,7-dioxo-5H-indolo[2,3-a]pyrrolo[3,4-c]carbazol wird durch Methylierung von 6,7,12,13-Tetrahydro-3,9-dimethoxy-6-methyl-5,7-dioxo-5H-indolo[2,3-a]pyrrolo[3,4-c]carbazol (Vorstufe für Beispiel 7) mit Methyljodid oder Dimethylsulfat wie folgt hergestellt: Eine Lösung von 1 g (2.5 mmol) 6,7,12,13-Tetrahydro-3,9-dimethoxy-6-methyl-5,7-dioxo-5H-indolo [2,3-a]pyrrolo[3,4-c]carbazol in 50 ml trockenem Dimethylformamid wird bei 20°C zu einer Suspension von 91 mg (3.0 mmol) Natriumhydrid (80%ig in Paraffinöl) in 5 ml Dimethylformamid getropft (Argonatmosphäre). Nach 1 Stunde Rühren bei 20°C werden 0.2 ml (3.2 mmol) Methyljodid zugegeben und 16 Stunden bei 20°C gerührt. Es wird im Vakuum eingedampft und der Rückstand zwischen Ethylacetat und Wasser verteilt. Die Ethylacetatphase wird abgetrennt, über Natriumsulfat getrocknet, filtriert und eingedampft. Der Rückstand wird mit Diisopropylether/Ethylacetat 2:1 (30 ml) ausgerührt und das nicht gelöste Produkt abfiltriert.
   Man erhält 6,7,12,13-Tetrahydro-3,9-dimethoxy-6,12-dimethyl-5,7-dioxo-5H-indolo[2,3-a]pyrrolo[3,4-c]carbazol als hellbraunes amorphes Pulver.
   In analoger Weise kann die Methylierung auch mit Dimethylsulfat als Alkylierungsmittel durchgeführt werden.
7c.) 6,7,12,13-Tetrahydro-12-methyl-5,7-dioxo-5H-indolo[2,3-a]pyrrolo[3,4-c]carbazol; Gelbe Kristalle vom Schmp. >340°C.
7d.) 6,7,12,13-Tetrahydro-3-methoxy-12-methyl-5,7-dioxo-5H-indolo[2,3-a]pyrrolo[3,4-c]carbazol; Orangefarbene Kristalle, die sich ab ca. 335°C zersetzen.
7e.) 6,7,12,13-Tetrahydro-12,13-dimethyl-5,7-dioxo-5H-indolo[2,3-a]pyrrolo[3,4-c]carbazol: Gelbe Kristalle vom Schmp. >330°C. Das als Ausgangsprodukt verwendete 6,7,12,13-Tetrahydro-6,12,13-trimethyl-5,7-dioxo-5H-indolo[2,3-a]pyrrolo[3,4-c]carbazol (Gelbe Kristalle, die sich ab 305°C zersetzen) wird durch Dimethylierung von 6,7,12,13-Tetrahydro-6-methyl-5,7-dioxo-5H-indolo[2,3-a]pyrrolo[3,4-c]carbazol (Vorstufe für Beispiel 5) mit zwei Äquivalenten Natriumhydrid und zwei Äquivalenten Methylierungsmittel, wie bei Beispiel 7b beschrieben, hergestellt, oder durch Trimethylierung des bekannten 6,7,12,13-Tetrahydro-5,7-dioxo-5H-indolo[2,3-a]pyrrolo[3,4-c]carbazol (Beispiel 11a) mit drei Äquivalenten Natriumhydrid und drei Äquivalenten Methylierungsmittel, ebenfalls analog wie bei Beispiel 7b beschrieben.
7f.) 6,7,12,13-Tetrahydro-12,13-diethyl-5,7-dioxo-5H-indolo[2,3-a]pyrrolo[3,4-c]carbazol; Hellgelbe Kristalle vom Schmp. 370°C. Herstellung analog Beispiel 7e, als Alkylierungsmittel wurde Ethylbromid verwendet.
7g.) 12-(3-Diisopropylaminopropyl)-6,7,12,13-tetrahydro-5,7-dioxo-5H-indolo[2,3-a]pyrrolo[3,4-c]carbazol; Gelbes amorphes Pulver vom Schmp. 225-227°C.
7h.) 12-(3-Diisopropylaminopropyl)-6,7,12,13-tetrahydro-13-methyl-5,7-dioxo-5H-indolo[2,3-a]pyrrolo[3,4-c]carbazol; Gelbe Kristalle vom Schmp. 268-270°C. Diese Verbindung wird analog zu Beispiel 7, ausgehend von 6,7,12,13-Tetrahydro-6,12-dimethyl-5,7-dioxo-5H-indolo[2,3-a]pyrrolo[3,4-c]carbazol, hergestellt. Die Vorstufe wird durch oxidative Cyclisierung von 3-(1-Methyl-3-indolyl)-4-(3-indolyl)-N-methylmaleinsäureimid mit DDQ und p-Toluolsulfonsäure Hydrat in Toluol, in analoger Weise wie bei Beispiel 5 für 3,4-Bis(indol-3-yl)-N-methylmaleinsäureimid beschrieben, hergestellt.
7i.) 6,7,12,13-Tetrahydro-5,7-dioxo-3,9-di-n-propoxy-5H-indolo[2,3-a]pyrrolo[3,4-c]carbazol; Orangefarbene Kristalle vom Schmp. >350°C. Die Verbindung wird analog zu Beispiel 7 ausgehend von 6,7,12,13-Tetrahydro-6-methyl-5,7-dioxo-3,9-di-n-propoxy-5H-indolo[2,3-a]pyrrolo[3,4-c]carbazol hergestellt. Die Vorstufe wird durch oxidative Cyclisierung von 3-(5-n-Propoxy-3-indolyl)-4-(5-n-propoxy-3-indolyl)-N-methylmaleinsäureimid mit DDQ und p-Toluolsulfonsäure Hydrat in Toluol, in analoger Weise wie bei Beispiel 5 für 3,4-Bis(indol-3-yl)-N-methylmaleinsäureimid beschrieben, hergestellt. 3-(5-n-Propoxy-3-indolyl)-4-(5-n-propoxy-3-indolyl)-N- methylmaleinsäureimid wird in Analogie zur Literatur (Tetrahedron 1988, 44, 2887) aus 3,4-Dibrom-N-methylmaleinsäureimid und 5-n-Propoxyindol hergestellt.

### Beispiel 8

### 12-(2,3-Dihydroxy-1-propyl)-6,7,12,13-tetrahydro-5,7-dioxo-5H-indolo[2,3-a]pyrrolo[3,4-c]carbazol

150 mg (0.34 mmol) 12-(2,2-Dimethyl-1,3-dioxolan-4-ylmethyl)-6,7,12,13-tetrahydro-5,7-dioxo-5H-indolo[2,3-a]pyrrolo[3,4-c]carbazol in 50 ml 1N Salzsäure/Ethanol 1:1 werden 16 Stunden bei 60°C gerührt. Nach dem Abkühlen wird mit 10%iger Kaliumhydrogencarbonatlösung neutralisiert und zweimal mit Ethylacetat (je 100 ml) extrahiert. Die Ethylacetatextrakte werden mit Wasser (100 ml) gewaschen, über Natriumsulfat getrocknet, filtriert und eingedampft. Der Rückstand wird an Kieselgel mit Toluol/Aceton 4:1 chromatographiert und die Fraktion mit dem Rf 0.2 in Toluol/Aceton 3:1 isoliert. Man erhält 12-(2,3-Dihydroxy-1-propyl)-6,7,12,13-tetrahydro-5,7-dioxo-5H-indolo[2,3-a]pyrrolo[3,4-c]carbazol in Form eines orangen amorphen Pulvers, das sich ab ca. 310°C zersetzt.

Das Ausgangsmaterial wird aus 12-(2,2-Dimethyl-1,3-dioxolan-4-ylmethyl)-6,7,12,13-tetrahydro-6-methyl-5,7-dioxo-5H-indolo[2,3-a]pyrrolo[3,4-c]carbazol durch Umsetzung mit 15%iger methanolischer Kaliumhydroxidlösung (16 stunden Rückfluß) analog Beispiel 6 zum 12-(2,2-Dimethyl-1,3-dioxolan-4-ylmethyl)-6,7,12,13-tetrahydro-5,7-dioxo-indolo[2,3-a]furano[3,4-c]carbazol und nachfolgender Umsetzung mit Hexamethyldisilazan und Methanol in Dimethylformamid analog Tetrahedron Lett. 1990, 5201 hergestellt.

12-(2,2-Dimethyl-1,3-dioxolan-4-ylmethyl)-6,7,12,13-tetrahydro-6-methyl-5,7-dioxo-5H-indolo[2,3-a]pyrrolo[3,4-c]carbazol wird wie folgt hergestellt:
3 g (8.84 mmol) 6,7,12,13-Tetrahydro-6-methyl-5,7-dioxo-5H-indolo[2,3-a]pyrrolo[3,4-c]carbazol (Vorstufe für Beispiel 5) werden mit 595 mg (9 mmol) gepulvertem Kaliumhydroxid, 620 mg (4.5 mmol) Kaliumcarbonat und 300 mg (1.1 mmol) 18-Crown-6 in 200 ml Toluol 2 Stunden bei 20°C gerührt (Stickstoffatmosphäre). Anschließend werden 2.6 g (9 mmol) 2,2-Dimethyl-4-(4-methylphenylsulfonyloxymethyl)-1,3-dioxolan (Biochemistry 1971, 10, 3204) zugefügt und es wird 16 Stunden unter Rückfluß erhitzt. Nach dem Abkühlen wird im Vakuum eingedampft und der Rückstand in Ethylacetat und Wasser aufgenommen. Die Ethylacetatlösung wird abgetrennt, mit Wasser gewaschen, über Natriumsulfat getrocknet, filtriert und eingedampft. Der Rückstand wird an Kieselgel mit Toluol/Aceton 9:1 chromatographiert, die Fraktion mit dem Rf 0.65 in Toluol/Aceton 3:1 isoliert, mit 20 ml Diisopropylether/Aceton 4:1 verrührt und die gebildeten gelben Kristalle von 12-(2,2-Dimethyl-1,3-dioxolan-4-yl methyl)-6,7,12,13-tetrahydro-6-methyl-5,7-dioxo-5H-indolo[2,3-a]pyrrolo[3,4-c]carbazol abfiltriert.

### Beispiel 9

### 12-(4-Aminobutyl)-6,7,12,13-tetrahydro-13-methyl-5,7-dioxo-5H-indolo[2,3-a]pyrrolo[3,4-c]carbazol

300 mg (0.7 mmol) 12-(4-Azidobutyl)-6,7,12,13-tetrahydro-13-methyl-5,7-dioxo-5H-indolo[2,3-a]pyrrolo[3,4-c]carbazol (Beispiel 1w) in 40 ml Tetrahydrofuran/Ethanol (1:1) werden mit 300 mg Palladium auf Aktivkohle (5%ig) als Katalysator bei 25°C und 50 bar Druck 48 Stunden im Autoklaven hydriert. Nach Filtration der Lösung wird das Lösungsmittel abdestilliert und der Rückstand aus Ethanol kristallisiert. Man erhält 12-(4-Aminobutyl)-6,7,12,13-tetrahydro-13-methyl-5,7-dioxo-5H-indolo[2,3-a]pyrrolo[3,4-c]carbazol in Form gelber Kristalle vom Schmp. 220-223°C.

In analoger Weise wird die folgende Verbindung erhalten:
9a.) 12-(5-Aminopentyl)-6,7,12,13-tetrahydro-13-methyl-5,7-dioxo-5H-indolo[2,3-a]pyrrolo[3,4-c]carbazol; Gelbe Kristalle vom Schmp. 177-181°C. (hergestellt aus Beispiel 1w).

### Beispiel 10

### (±)-12-(3-Dimethylamino-2-hydroxy-1-propyl)-6,7,12,13-tetrahydro-13-methyl-5,7-dioxo-5H-indolo[2,3-a]pyrrolo[3,4-c]-carbazol 750 mg (1.7 mmol)

(±)-12-(3-Dimethylamino-2-hydroxy-1-propyl)-6,7,12,13-tetrahydro-13-methyl-5,7-dioxo-indolo[2,3-a]furano[3,4-c]carbazol werden im Autoklaven mit 50 ml ammoniakgesättigtem Ethanol 20 Stunden auf 140°C erhitzt. Das ungelöste Produkt wird abfiltriert und erneut aus Ethanol kristallisiert. Man erhält (±)-12-(3-Dimethylamino-2-hydroxy-1-propyl)-6,7,12,13-tetrahydro-13-methyl-5,7-dioxo-5H-indolo[2,3-a]pyrrolo[3,4-c]carbazol als gelbes amorphes Pulver vom Schmp. 260-270°C.

Das Ausgangsprodukt wird wie folgt hergestellt:
950 mg (2.1 mmol) (±)-12-(3-Dimethylamino-2-hydroxy-1-propyl)-6,7,12,13-tetrahydro-6,13-dimethyl-5,7-dioxo-5H-indolo[2,3-a]pyrrolo[3,4-c]carbazol werden in 150 ml 15%iger methanolischer Kaliumhydroxidlösung 16 Stunden unter Rückfluß erhitzt. Die Losung wird zur Hälfte eingedampft, mit 100 ml Wasser versetzt und mit halbkonzentrierter Salzsäure schwach angesäuert. Der ausgefallene Niederschlag wird abfiltiert, mit 5%iger Natriumhydrogencarbonatlösung (50 ml) verrührt, erneut filtriert und getrocknet. Man erhält (±)-12-(3-Dimethylamino-2-hydroxy-1-propyl)-6,7,12,13-tetrahydro-13-methyl-5,7-dioxo-indolo[2,3-a]furano[3,4-c]carbazol als gelbes amorphes Pulver.

Das Ausgangsprodukt wird wie folgt hergestellt: 950 mg (2.3 mmol) (±)-12-Epoxymethyl-6,7,12,13-tetrahydro-6,13-dimethyl-5,7-dioxo-5H-indolo[2,3-a]pyrrolo[3,4-c]carbazol werden in 150 ml einer 33%igen Dimethylaminlösung in Ethanol 2 Stunden auf 50°C erhitzt. Nach dem Abkühlen wird die Lösung eingedampft, der Rückstand mit 25 ml Diisopropylether/Aceton 4:1 verrührt und das Produkt abfiltriert. Man erhält (±)-12-(3-Dimethyl-amino-2-hydroxy-1-propyl)-6,7,12,13-tetrahydro-6,13-dimethyl-5,7-dioxo-5H-indolo[2,3-a]pyrrolo[3,4-c]carbazol als gelbes Pulver vom Schmp. 198-203°C.

Das Ausgangsprodukt wird wie folgt hergestellt: 3.9 g (9.5 mmol) (±)-3-(1-Epoxymethyl-3-indolyl)-4-(1-methyl-3-indolyl)-N-methylmaleimid, 2.16 g (11.4 mmol) p-Toluolsulfonsäure Hydrat und 4.2 g (19 mmol) DDQ werden in 1,5 l Toluol 15 stunden unter Rückfluß erhitzt. Es wird im Vakuum eingedampft, der Rückstand mit 300 ml 1N Natronlauge im Ultraschallbad behandelt, mit Kochsalz gesättigt und zweimal mit je 300 ml Tetrahydrofuran extrahiert. Die THF-Lösungen werden zweimal mit je 300 ml gesättigter Kochsalzlösung gewaschen, getrocknet (Natriumsultat) und eingedampft. Der Rückstand wird an Kieselgel mit Dichlormethan als Laufmittel chromatographiert.
Die Fraktion mit dem Rf 0.7 in Toluol/Aceton 3:1 wird isoliert, mit Diisopropylether/Aceton 4:1 (20 ml) verrührt und das nicht gelöste Produkt abfiltriert. Man erhält (±)-12-Epoxymethyl-6,7,12,13-tetrahydro-6,13-dimethyl-5,7-dioxo-5H-indolo[2,3-a]pyrrolo[3,4-c]carbazol als gelbes amorphes Pulver. Das Ausgangsprodukt wird wie folgt hergestellt: 4.0 g (11.2 mmol) 3-(1-Methyl-3-indolyl)-4-(3-indolyl)-N-methyl-maleinsäureimid in 100 ml trockenem Dimethylformamid unter Stickstoffatmosphäre werden portionsweise bei 0°C mit 405 mg (13.5 mmol) Natriumhydrid (80%ig in Mineralöl) versetzt. Nach einer Stunde Rühren bei 0°C werden 3,1 g (22.6 mmol) Epibromhydrin in 10 ml DMF zugetropft und anschließend 16 Stunden bei 20°C gerührt.
Die Lösung wird mit 400 ml gesättigter Kochsalzlösung und 400 ml Tetrahydrofuran versetzt, die organische Phase abgetrennt und die wässrige Phase erneut mit 400 ml Tetrahydrofuran extrahiert. Die organischen Phasen werden mit gesättigter Kochsalzlösung gewaschen (zweimal 400 ml), über Natriumsulfat getrocknet, filtriert und eingedampft. Der Rückstand wird an Kieselgel mit Dichlormethan chromatographiert. Die Fraktion mit dem Rf 0.6 in Toluol/Aceton 3:1 wird isoliert. Man erhält (±)-3-(1-Epoxymethyl-3-indolyl)-4-(1-methyl-3-indolyl)-N-methylmaleinsäureimid als rote amorphe Substanz. Das Ausgangsprodukt wird durch Umsetzung von 3-Brom-4-(1-methyl-3-indolyl)-N-methylmaleinsäureimid mit Indolylmagnesiumbromid in Analogie zur Literatur (Tetrahedron 1988, 44, 2987) hergestellt.

3-Brom-4-(1-methyl-3-indolyl)-N-methylmaleinsäureimid wird wie folgt hergestellt:
15g (49.2 mmol) 3-Brom-4-(3-indolyl)-N-methylmaleinsäureimid (Tetrahedron 1988, 44, 2887) in 330 ml trockenem Tetrahydrofuran unter Stickstoffatmosphäre werden portionsweise bei 4°C mit 1.5 g (50 mmol) Natriumhydrid (80%ig in Mineralöl) versetzt. Nach einer Stunde Rühren bei 4°C werden 7.5 g (53 mmol) Methyljodid in 35 ml THF zugetropft und anschließend 15 Stunden bei 20°C gerührt. Die Lösung wird zweimal mit je 300 ml Kochsalzlösung gewaschen, über Natriumsulfat getrocknet, filtriert und eingedampft. Der Rückstand wird aus 100 ml Methanol kristallisiert. Man erhält 3-Brom-4-(1-methyl-3-indolyl)-N-methyl-maleinsäureimid als orangefarbenes Pulver.

In analoger Weise werden die folgenden Verbindungen erhalten:
10a.) (±)-12-(3-Diethylamino-2-hydroxy-1-propyl)-6,7,12,13-tetrahydro-13-methyl-5,7-dioxo-5H-indolo[2,3-a]pyrrolo[3,4-c]carbazol; Gelbes amorphes Pulver, Erweichung ab ca. 185°C bis 260°C.
10b.) (±)-13-(3-Dimethylamino-2-hydroxy-1-propyl)-6,7,12,13-tetrahydro-3-methoxy-12-methyl-5,7-dioxo-5H-indolo[2,3-a]pyrrolo[3,4-c]carbazol; Gelbes amorphes Pulver vom Schmp. 235-240°C.
10c.) (±)-12-(3-Dimethylamino-2-hydroxy-1-propyl)-6,7,12,13-tetrahydro-3-methoxy-13-methyl-5,7-dioxo-5H-indolo[2,3-a]pyrrolo[3,4-c]carbazol; Gelbes amorphes Pulver vom Schmp. 240-250°C.
10d.) (±)-12-(3-Diisopropylamino-2-hydroxy-1-propyl)-6,7,12,13-tetrahydro-13-methyl-5,7-dioxo-5H-indolo[2,3-a]pyrrolo[3,4-c]carbazol; Gelbes amorphes Pulver vom Schmp. 256-260°C.
10e.) (±)-12-(3-Pyrrolidino-2-hydroxy-1-propyl)-6,7,12,13-tetrahydro-13-methyl-5,7-dioxo-5H-indolo[2,3-a]pyrrolo[3,4-c]carbazol; Gelbes amorphes Pulver vom Schmp. 130-135°C.

### Beispiel 11

Für die folgenden bekannten (Beispiele 11a - 11h), nach Literaturverfahren oder in Analogie zu den Literaturverfahren, hergestellten, an den Indolstickstoffatomen unsubstituierten, Indolocarbazolderivate der allgemeinen Formel Ia wurden überraschenderweise ebenfalls inhibitorische Wirkungen für Proteinkinasen, insbesondere der Proteinkinase C und/oder der Myosin Light-Chain Kinase gefunden.
11a.) 6,7,12,13-Tetrahydro-5,7-dioxo-5H-indolo[2,3-a]pyrrolo[3,4-c]carbazol; Gelbe Kristalle vom Schmp. >370°C
11b.) 6,7,12,13-Tetrahydro-3-methoxy-5,7-dioxo-5H-indolo[2,3-a]pyrrolo[3,4-c]carbazol; Dunkelgelbe Kristalle vom Schmp. >330°C
11c.) 2-Chlor-6,7,12,13-tetrahydro-5,7-dioxo-5H-indolo[2,3-a]pyrrolo[3,4-c]carbazol: Hellgelbe Kristalle vom Schmp. >350°C.
11d.) 6,7,12,13-Tetrahydro-3,9-dimethoxy-5,7-dioxo-5H-indolo[2,3-a]pyrrolo[3,4-c]carbazol: Gelbe Kristalle vom Schmp. >300°C.
11e.) 6,7,12,13-Tetrahydro-3,9-dimethyl-5,7-dioxo-5H-indolo[2,3-a]pyrrolo[3,4-c]carbazol; Orange Kristalle vom Schmp. >300°C.
11f.) 3,9-Dichlor-6,7,12,13-tetrahydro-5,7-dioxo-5H-indolo[2,3-a]pyrrolo[3,4-c]carbazol; Gelbe Kristalle von Schmp. >300°C.
11g.) 3,9-Dibrom-6,7,12,13-tetrahydro-5,7-dioxo-5H-indolo[2,3-a]pyrrolo[3,4-c]carbazol; Gelbe Kristalle vom Schmp. >300°C.
11h.) 1,11-Dichlor-6,7,12,13-tetrahydro-5,7-dioxo-5H-indolo-[2,3-a]pyrrolo[3,4-c]carbazol; Dunkelgelbe Kristalle vom Schmp. >340°C.

## Patentansprüche

1. Indolocarbazol-Imide der allgemeinen Formel I, 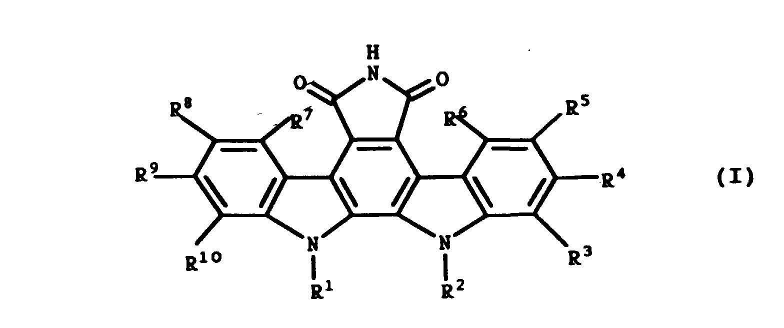 dadurch gekennzeichnet, daß R¹ und R² gleich oder verschieden sind und Wasserstoff, Methyl, Ethyl, Cyanomethyl, 2-Cyanoethyl, 3-Cyanopropyl, 3-Cyano-2-propyl, 2-Azidoethyl, 3-Azidopropyl, 2,3-Dihydroxypropyl, 3-Chlor-2-hydroxy-1-propyl, 3-Aminopropyl, 3-Dimethylaminopropyl, 3-Trimethylammoniopropyl, 4-Aminobutyl, 5-Aminopentyl, Epoxymethyl, 2-Epoxyethyl, 3-Methylaminopropyl, 3-Ethylaminopropyl, 3-Isopropylaminopropyl, 3-Diisopropylaminopropyl, 3-Methylamino-2-hydroxy-1-propyl, 3-Ethylamino-2-hydroxy-1-propyl, 3-Isopropylamino-2-hydroxy-1-propyl, 3-Dimethylamino-2-hydroxy-1-propyl, 3-Diethylamino-2-hydroxy-1-propyl, 3-Diisopropylamino-2-hydroxy-1-propyl, 3-Pyrroiidino-2-hydroxy-1-propyl oder einen Rest -(CH₂)₂CONH₂ bedeuten und R³ bis R¹⁰ unabhängig voneinander für Wasserstoff, Methyl, Methoxy, n-Propoxy, Chlor, Brom, Nitro, Hydroxy, Amino oder zwei benachbarte Reste zusammen für Methylendioxy stehen, mit den Maßgaben, daß
(1.) nicht alle Reste R¹ bis R¹⁰ gleichzeitig Wasserstoff sein dürfen und daß
(2.) alle übrigen Reste nicht gleichzeitig Wasserstoff sein dürfen, wenn (a) R¹ eine 3-Dimethylaminopropylgruppe ist, oder wenn (b) R³ und R¹⁰ gleichzeitig Chlor, Hydroxy oder Methoxy bedeuten, oder wenn (c) R⁴ und/oder R⁹ Chlor, Hydroxy oder Methoxy bedeuten, oder wenn (d) R⁵ und/oder R⁸ Methyl, Methoxy, Benzyloxy, Chlor, Brom oder Fluor bedeuten, sowie deren pharmakologisch unbedenklichen Salze.

2. Indolocarbazol-Imide der allgemeinen Formel I gemäß Anspruch 1, nämlich
13-(2-Cyanoethyl)-6,7,12,13-tetrahydro-3-methoxy-12-methyl-5,7-dioxo-5H-indolo[2,3-a]pyrrolo[3,4-c]carbazol;
12-(2-Cyanothyl)-6,7,12,13-tetrahydro-13-methyl-5,7-dioxo-5H-indolo[2,3-a]pyrrolo[3,4-c]carbazol;
12-(2-Cyanoethyl)-6,7,12,13-tetrahydro-3-methoxy-13-methyl-5,7-dioxo-5H-indolo[2,3-a]pyrrolo[3,4-c]carbazol;
12-(2-Cyanoethyl)-6,7,12,13-tetrahydro-3,9-dimethoxy-13-methyl-5,7-dioxo-5H-indolo[2,3-a]pyrrolo[3,4-c]carbazol;
12-(3-Cyano-2-propyl)-6,7,12,13-tetrahydro-13-methyl-5,7-dioxo-5H-indolo[2,3-a]pyrrolo[3,4-c]carbazol;
12-Cyanomethyl-6,7,12,13-tetrahydro-13-methyl-5,7-dioxo-5H-indolo[2,3-a]pyrrolo[3,4-c]carbazol;
3-Chlor-13-(2-cyanoethyl)-6,7,12,13-tetrahydro-12-methyl-5,7-dioxo-5H-indolo[2,3-a]pyrrolo[3,4-c]carbazol;
12-(3-Cyanopropyl)-6,7,12,13-tetrahydro-13-methyl-5,7-dioxo-5H-indolo[2,3-a]pyrrolo[3,4-c]carbazol;
12-(3-Cyanopropyl)-6,7,12,13-tetrahydro-3-methoxy-13-methyl-5,7-dioxo-5H-indolo[2,3-a]pyrrolo[3,4-c]carbazol;
12-[3-(4-Methylphenylsulfonyloxy)propyl]-6,7,12,13-tetrahydro-13-methyl-5,7-dioxo-5H-indolo[2,3-a]pyrrolo[3,4-c]carbazol;
12-(3-Dimethylaminopropyl)-6,7,12,13-tetrahydro-13-methyl-5,7-dioxo-5H-indolo[2,3-a]pyrrolo[3,4-c]carbazol;
13-(3-Dimethylaminopropyl)-6,7,12,13-tetrahydro-3-methoxy-12-methyl-5,7-dioxo-5H-indolo[2,3-a]pyrrolo[3,4-c]carbazol;
12-(3-Azidopropyl)-6,7,12,13-tetrahydro-13-methyl-5,7-dioxo-5H-indolo[2,3-a]pyrrolo[3,4-c]carbazol;
12-(2-Azidoethyl)-6,7,12,13-tetrahydro-13-methyl-5,7-dioxo-5H-indolo[2,3-a]pyrrolo[3,4-c]carbazol;
3-Chlor-13-(2-cyanoethyl)-6,7,12,13-tetrahydro-9-methoxy-12-methyl-5,7-dioxo-5H-indolo[2,3-a]pyrrolo[3,4-c]carbazol;
13-(2-Cyanoethyl)-6,7,12,13-tetrahydro-2,3-dimethoxy-12-methyl-5,7-dioxo-5H-indolo[2,3-a]pyrrolo[3,4-c]carbazol;
13-(2-Cyanoethyl)-6,7,12,13-tetrahydro-12-methyl-5,7-dioxo-3-n-propoxy-5H-indolo[2,3-a]pyrrolo[3,4-c]carbazol;
13-(3-Cyanopropyl)-6,7,12,13-tetrahydro-3-methoxy-12-methyl-5,7-dioxo-5H-indolo[2,3-a]pyrrolo[3,4-c]carbazol;
12-(3-Cyanopropyl)-6,7,12,13-tetrahydro-3,9-dimethoxy-13-methyl-5,7-dioxo-5H-indolo[2,3-a]pyrrolo[3,4-c]carbazol;
13-(2-Cyanoethyl)-6,7,12,13-tetrahydro-2,3,9-trimethoxy-12-methyl-5,7-dioxo-5H-indolo[2,3-a]pyrrolo[3,4-c]carbazol;
13-(2-Cyanoethyl)-6,7,12,13-tetrahydro-1-methoxy-12-methyl-5,7-dioxo-5H-indolo[2,3-a]pyrrolo[3,4-c]carbazol;
13-(2-Cyanoethyl)-6,7,12,13-tetrahydro-2-methoxy-12-methyl-5,7-dioxo-5H-indolo[2,3-a]pyrrolo[3,4-c]carbazol;
12-(4-Azidobutyl)-6,7,12,13-tetrahydro-13-methyl-5,7-dioxo-5H-indolo[2,3-a]pyrrolo[3,4-c]carbazol;
12-(5-Azidopentyl)-6,7,12,13-tetrahydro-13-methyl-5,7-dioxo-5H-indolo[2,3-a]pyrrolo[3,4-c]carbazol;
12-(2-Dimethylaminoethyl)-6,7,12,13-tetrahydro-13-methyl-5,7-dioxo-5H-indolo[2,3-a]pyrrolo[3,4-c]carbazol;
12-(3-Dimethylaminopropyl)-6,7,12,13-tetrahydro-3-methoxy-13-methyl-5,7-dioxo-5H-indolo[2,3-a]pyrrolo[3,4-c]carbazol;
13-(3-Dimethylaminopropyl)-6,7,12,13-tetrahydro-2,3-dimethoxy-12-methyl-5,7-dioxo-5H-indolo[2,3-a]pyrrolo[3,4-c]carbazol;
12-Epoxymethyl-6,7,12,13-tetrahydro-13-methyl-5,7-dioxo-5H-indolo[2,3-a]pyrrolo[3,4-c]carbazol; und
(±)-12-(3-Chlor-2-hydroxy-1-propyl)-6,7,12,13-tetrahydro-13-methyl-5,7-dioxo-5H-indolo[2,3-a]pyrrolo[3,4-c]carbazol.

3. Indolocarbazol-Imide der allgemeinen Formel I gemäß Anspruch 1, nämlich
13-(2-Cyanoethyl)-6,7,12,13-tetrahydro-3-hydroxy-12-methyl-5,7-dioxo-5H-indolo[2,3-a]pyrrolo[3,4-c]carbazol;
12-(2-Cyanoethyl)-6,7,12,13-tetrahydro-3-hydroxy-13-methyl-5,7-dioxo-5H-indolo[2,3-a]pyrrolo[3,4-c]carbazol;
13-(2-Cyanoethyl)-6,7,12,13-tetrahydro-2,3-dihydroxy-12-methyl-5,7-dioxo-5H-indolo[2,3-a]pyrrolo[3,4-c]carbazol;
12-(3-Dimethylaminopropyl)-6,7,12,13-tetrahydro-3,9-dihydroxy-5,7-dioxo-5H-indolo[2,3-a]pyrrolo[3,4-c]carbazol;
12-(3-Dimethylaminopropyl)-6,7,12,13-tetrahydro-3(9)-hydroxy-9(3)-methoxy-5,7-dioxo-5H-indolo[2,3-a]pyrrolo[3,4-c]carbazol;
6,7,12,13-Tetrahydro-3,9-dihydroxy-5,7-dioxo-5H-indolo[2,3-a]pyrrolo[3,4-c]carbazol;
12-(2-Cyanoethyl)-6,7,12,13-tetrahydro-3,9-dihydroxy-13-methyl-5,7-dioxo-5H-indolo[2,3-a]pyrrolo[3,4-c]carbazol;
13-(3-Dimethylaminopropyl)-6,7,12,13-tetrahydro-3-hydroxy-12-methyl-5,7-dioxo-5H-indolo[2,3-a]pyrrolo[3,4-c]carbazol; und
12-(3-Diisopropylaminopropyl)-6,7,12,13-tetrahydro-3-hydroxy-13-methyl-5,7-dioxo-5H-indolo[2,3-a]pyrrolo[3,4-c]carbazol.

4. Indolocarbazol-Imid der allgemeinen Formel I gemäß Anspruch 1
12-(3-Dimethylaminopropyl)-6,7,12,13-tetrahydro-13-methyl-5,7-dioxo-5H-indolo[2,3-a]pyrrolo[3,4-c]carbazol Methojodid.

5. Indolocarbazol-Imide der allgemeinen Formel I gemäß Anspruch 1, nämlich
13-(3-Aminopropyl)-6,7,12,13-tetrahydro-3-methoxy-12-methyl-5,7-dioxo-5H-indolo[2,3-a]pyrrolo[3,4-c]carbazol;
13-(3-Aminopropyl)-3-chlor-6,7,12,13-tetrahydro-9-methoxy-12-methyl-5,7-dioxo-5H-indolo[2,3-a]pyrrolo[3,4-c]carbazol; und
13-(3-Isopropylaminopropyl)-6,7,12,13-tetrahydro-13-methyl-5,7-dioxo-5H-indolo[2,3-a]pyrrolo[3,4-c]carbazol.

6. Indolocarbazolimid der allgemeinen Formel I gem. Anspruch 1,
12-(2-Carbamoylethyl)-6,7,12,13-tetrahydro-5,7-dioxo-5H-indolo[2,3-a]pyrrolo[3,4-c]carbazol.

7. Indolocarbazolimid der allgemeinen Formel I gem. Anspruch 1
(±)-12-(3-Diethylamino-2-hydroxy-1-propyl)-6,7,12,13-tetrahydro-5,7-dioxo-5H-indolo[2,3-a]pyrrolo[3,4-c]carbazol.

8. Indolocarbazol-Imide der allgemeinen Formel I gem. Anspruch 1, nämlich
12-(3-Dimethylaminopropyl)-6,7,12,13-tetrahydro-3,9-dimethoxy-5,7-dioxo-5H-indolo[2,3-a]pyrrolo[3,4-c]carbazol;
6,7,12,13-Tetrahydro-3,9-dimethoxy-12-methyl-5,7-dioxo-5H-indolo[2,3-a]pyrrolo[3,4-c]carbazol;
6,7,12,13-Tetrahydro-12-methyl-5,7-dioxo-5H-indolo[2,3-a]pyrrolo[3,4-c]carbazol;
6,7,12,13-Tetrahydro-3-methoxy-12-methyl-5,7-dioxo-5H-indolo[2,3-a]pyrrolo[3,4-c]carbazol;
6,7,12,13-Tetrahydro-12,13-dimethyl-5,7-dioxo-5H-indolo[2,3-a]pyrrolo[3,4-c]carbazol;
6,7,12,13-Tetrahydro-12,13-diethyl-5,7-dioxo-5H-indolo[2,3-a]pyrrolo[3,4-c]carbazol;
12-(3-Diisopropylaminopropyl)-6,7,12,13-tetrahydro-5,7-dioxo-5H-indolo[2,3-a]pyrrolo[3,4-c]carbazol;
12-(3-Diisopropylaminopropyl)-6,7,12,13-tetrahydro-13-methyl-5,7-dioxo-5H-indolo[2,3-a]pyrrolo[3,4-c]carbazol; und
6,7,12,13-Tetrahydro-5,7-dioxo-3,9-di-n-propoxy-5H-indolo[2,3-a]pyrrolo[3,4-c]carbazol.

9. Indolocarbazolimide der allgemeinen Formel I gem. Anspruch 1,
12-(2,3-Dihydroxy-1-propyl)-6,7,12,13-tetrahydro-5,7-dioxo-5H-indolo[2,3-a]pyrrolo[3,4-c]carbazol.

10. Indolocarbazol-Imide der allgemeinen Formel I gemäß Anspruch 1, nämlich
12-(4-Aminobutyl)-6,7,12,13-tetrahydro-13-methyl-5,7-dioxo-5H-indolo[2,3-a]pyrrolo[3,4-c]carbazol und
12-(5-Aminopentyl)-6,7,12,13-tetrahydro-13-methyl-5,7-dioxo-5H-indolo[2,3-a]pyrrolo[3,4-c]carbazol.

11. Indolocarbazol-Imide der allgemeinen Formel I gem. Anspruch 1, nämlich
(±)-12-(3-Dimethylamino-2-hydroxy-1-propyl)-6,7,12,13-tetrahydro-13-methyl-5,7-dioxo-5H-indolo[2,3-a]pyrrolo[3,4-c]-carbazol;
(±)-12-(3-Diethylamino-2-hydroxy-1-propyl)-6,7,12,13-tetrahydro-13-methyl-5,7-dioxo-5H-indolo[2,3-a]pyrrolo[3,4-c]carbazol;
(±)-13-(3-Dimethylamino-2-hydroxy-1-propyl)-6,7,12,13-tetrahydro-3-methoxy-12-methyl-5,7-dioxo-5H-indolo[2,3-a]pyrrolo[3,4-c]carbazol;
(±)-12-(3-Dimethylamino-2-hydroxy-1-propyl)-6,7,12,13-tetrahydro-3-methoxy-13-methyl-5,7-dioxo-5H-indolo[2,3-a]pyrrolo[3,4-c]carbazol;
(±)-12-(3-Diisopropylamino-2-hydroxy-1-propyl)-6,7,12,13-tetrahydro-13-methyl-5,7-dioxo-5H-indolo[2,3-a]pyrrolo[3,4-c]carbazol; und
(±)-12-(3-Pyrrolidino-2-hydroxy-1-propyl)-6,7,12,13-tetrahydro-13-methyl-5,7-dioxo-5H-indolo[2,3-a]pyrrolo[3,4-c]carbazol.

12. Verfahren zur Herstellung von Verbindungen I gemäß der Ansprüche 1 bis 11, dadurch gekennzeichnet, daß
A) durch oxidative Cyclisierung von Bisindolylmaleimiden II, bei denen R¹ bis R¹⁰ eine der oben genannten Bedeutungen besitzen und R¹³ für Wasserstoff, Methyl oder eine leicht abspaltbare Schutzgruppe Z steht, zu Verbindungen I bzw. Verbindungen III, 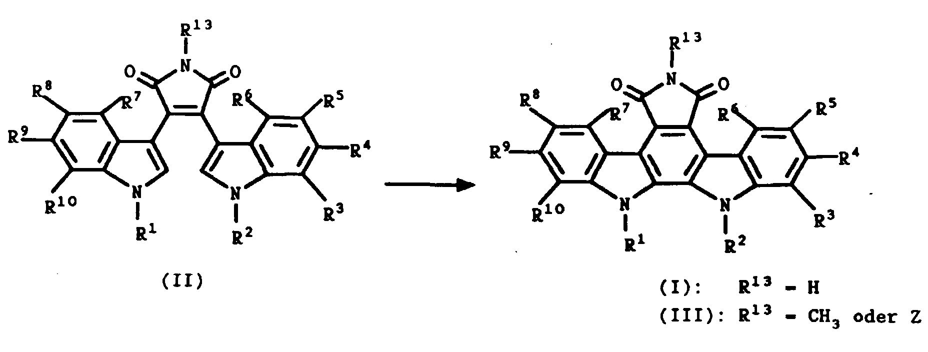 umgesetzt wird, wobei
(a) Verbindungen III, bei denen R¹ und/oder R² einen unsubstituierten, N-mono- oder N,N-disubstituierten 3-Amino-2-hydroxy-1-propylrest bedeuten, durch Umsetzung von Verbindungen III, bei denen die entsprechenden Reste R¹ und/oder R² für einen Epoxymethyl- oder 2-Epoxyethylrest stehen, mit Ammoniak oder Aminen der allgemeinen Formel HNRR', in der R und R' für Wasserstoff oder eine C₁₋₄-Alkylgruppe stehen oder in der R und R' zusammen mit dem Stickstoffatom einen heterocyclischen Ring mit 3 bis 6 C-Atomen bilden, der auch Sauerstoff-, Schwefel und/oder weitere Stickstoffatome enthalten und durch C₁₋₄-Alkylreste substituiert sein kann, hergestellt werden;
(b) Verbindungen III, bei denen R¹³ für eine Methylgruppe steht, durch Umsetzung mit Kaliumhydroxid in Wasser oder Alkoholen in die entsprechenden Anhydride und diese durch Umsetzung mit Ammoniak, Ammoniumacetat oder mit Hexamethyldisilazan und Methanol in Dimethylformamid in an sich bekannter Weise in die entsprechenden unsubstituierten Imide I übergeführt werden; oder
(c) Verbindungen III, bei denen R¹³ für eine Schutzgruppe Z steht, durch geeignete Abspaltung der Schutzgruppe Z in die entsprechenden unsubstituierten Imide I übergeführt werden;
B) durch Substitution von Indolocarbazolen der allgemeinen Formeln Ia oder IIIa, 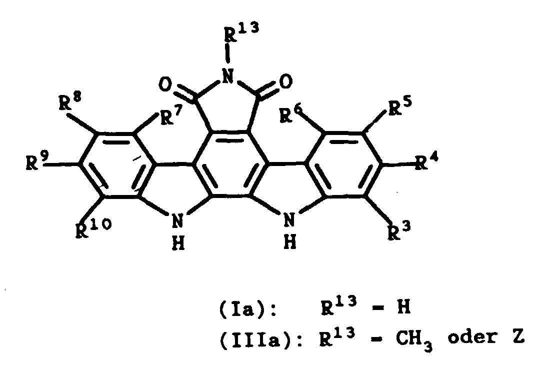 in denen R¹ und R² für Wasserstoff stehen, an einem oder an beiden Indolstickstoffatomen, z.B. durch Alkylierung mit einer Verbindung IV,
R¹⁴-X (IV)
in der R¹⁴ eine der für R¹ und R² angegebenen Bedeutungen besitzt und X eine geeignete Abgangegruppe, wie z.B. Chlor, Brom, Jod oder Tosyl darstellt, unter für die Alkylierung von Indolen bzw. Carbazolen geeigneten, bekannten Reaktionsbedingungen, Verbindungen I oder III hergestellt werden; wobei
(a) an den Indolstickstoffatomen monosubstituierte Verbindungen der allgemeinen Formeln I oder III, in denen einer der Reste R¹ oder R² für Wasserstoff steht, durch eine weitere Substitution in an den Indolstickstoffatomen disubstituierte Verbindungen der allgemeinen Formeln I oder III übergeführt werden können, in denen R¹ und R² verschiedene Bedeutungen besitzen;
(b) Verbindungen der allgemeinen Formel III anschließend, wie bei Verfahren A beschrieben, in Verbindungen der allgemeinen Formel I übergeführt werden;
(c) Verbindungen der allgemeinen Formeln I oder III, in denen R¹ und/oder R² für einen 2-Cyanoethyl- oder 3-Cyano-2-propyl-steht, durch basenkatalysierte Michaeladdition von Verbindungen der allgemeinen Formeln I oder III, in denen R¹ und/oder R² für Wasserstoff steht, an aktivierte Olefine der allgemeinen Formel V,
R¹⁵-CH=CH-R¹⁶ (V)
in der R¹⁵ für Wasserstoff oder Methyl und R¹⁶ für Cyano, oder R¹⁵ für Wasserstoff und R¹⁶ für einen Alkoxycarbonylrest mit bis zu 5 C-Atomen oder für CONH₂ steht, hergestellt werden;
(d) Verbindungen der allgemeinen Formel I oder III, in denen R¹ und/oder R² für einen N,N-disubstituierten 3-Amino-2-hydroxy-1-propylrest steht, durch Alkylierung von Verbindungen der allgemeinen Formeln I oder III, in denen R¹ und/oder R² für Wasserstoff steht, mit 1,1-disubstituierten 3-Hydroxyazetidiniumhalogeniden hergestellt werden;
(f) Verbindungen der allgemeinen Formeln I oder III, in denen R¹ und R² zusammen eine Alkylengruppe mit 2 bis 4-C-Atomen bilden, durch Alkylierung von Verbindungen der allgemeinen Formeln Ia oder IIIa, in denen R¹ und R² für Wasserstoff stehen, mit 2 Äquivalenten einer Base und eines Dihalogenalkan hergestellt werden;
(g) Verbindungen der allgemeinen Formel I oder III, in denen R¹ und/oder R² für einen Methyl- oder Ethylrest stehen, auch durch Alkylierung mit Dimethyl- oder Diethylsulfat in bekannter Weise hergestellt werden;
(h) Verbindungen der allgemeinen Formeln I und III, in denen R¹ und/oder R² für eine Cyanogruppe stehen, durch Umsetzung von Verbindungen der allgemeinen Formeln I oder III, in denen R¹ und/oder R² für Wasserstoff stehen, mit Natriumhydrid und Phenylcyanat in Dimethylformamid hergestellt werden;
(i) bei Verbindungen der allgemeinen Formeln I oder III, in denen einer oder beide der Reste R¹ und/oder R² durch Umsetzung mit einem Alkylierungsmittel der allgemeinen Formel IV eingeführt wurde, die eingeführten Reste R¹ und/oder R² anschließend durch Hydrolyse, Etherspaltung, Amidbildung oder Reduktion modifiziert werden können, so daß sie eine andere der für R¹ und R² genannten Bedeutungen erhalten;
(j) Verbindungen der allgemeinen Formeln I oder III, in denen R¹ und/oder R² für einen Hydroxyalkylrest stehen, auch durch Hydrolyse von Verbindungen der allgemeinen Formeln I oder III, in denen R¹ und/oder R² für einen Halogenalkylrest stehen, oder durch Etherspaltung von Verbindungen der allgemeinen Formeln I oder III, in denen R¹ und/oder R² für einen Alkoxyalkylrest stehen, oder durch Umsetzung von Verbindungen der allgemeinen Formeln I oder III, in denen R¹ und/oder R² für Wasserstoff stehen, mit einen Alkylenoxid, z.B. Propylenoxid, hergestellt werden;
(l) Verbindungen der allgemeinen Formeln I oder III, in denen R¹ und/oder R² für einen N-unsubstituierten Aminoalkylrest stehen, vorzugsweise durch katalytische Hydrierung von Verbindungen der allgemeinen Formeln I oder III, in denen R¹ und/oder R² für einen Cyanoalkyl- oder einen Azidoalkylrest stehen, hergestellt werden;
(m) Verbindungen der allgemeinen Formeln I oder III, in denen R¹ und/oder R² für einen Amidinoalkylrest stehen, auch nach bekannten Methoden aus Verbindungen der allgemeinen Formeln I oder III, in denen R¹ und/oder R² für einen Cyanoalkylrest oder einen Carboxyalkylrest stehen, hergestellt werden;
(n) N-Unsubstituierte Aminoalkylgruppen R¹ und/oder R² in Verbindungen der allgemeinen Formeln I oder III nach bekannten Methoden alkyliert oder acyliert werden können, und gegebenenfalls nach weiteren bekannten Methoden in andere funktionelle Modifikationen einer unsubstituierten Aminoalkylgruppe übergeführt werden können;
(o) Hydroxyalkylgruppen R¹ und/oder R² in Verbindungen der allgemeinen Formeln I oder III nach bekannten Methoden funktionell modifiziert werden können, z.B. durch Acylierung zu Acyloxyalkylgruppen, zu Halogenalkylgruppen oder durch Umsetzung mit Arylsulfonsäurechloriden zu Arylsulfonyloxyalkylgruppen;
(p) Arylsulfonyloxyalkylgruppen und Alkylsulfonyloxyalkylgruppen R¹ und/oder R² in Verbindungen der allgemeinen Formeln I oder III in bekannter Weise durch Umsetzung mit Kaliumcyanid in Cyanoalkylgruppen, durch Umsetzung mit Natriumazid in Azidoalkylgruppen, durch Umsetzung mit Aminen in Aminoalkylgruppen oder durch Umsetzung mit Thioharnstoff in Amidinothioalkylgruppen übergeführt werden können;
(q) Alkylthioalkylgruppen R¹ und/oder R² in Verbindungen der allgemeinen Formeln I oder III zu Alkylsulfinylalkylgruppen oder Alkylsulfonylalkylgruppen oxidiert werden können; oder auch
C) wie für die Reste R¹ und/oder R² in Verbindungen der allgemeinen Formeln I oder III beschrieben, die Reste R³ bis R¹⁰ in Verbindungen der allgemeinen Formeln I oder III funktionell modifiziert werden, wobei
(a) Reste R³ bis R¹⁰, die für Nitro stehen, durch bekannte Methoden zu Resten R³ bis R¹⁰, die für Amino stehen, reduziert werden können;
(b) Reste R³ bis R¹⁰, die für Methoxy stehen, durch Etherspaltung, oder Reste R³ bis R¹⁰, die für Benzyloxy stehen, durch katalytische Hydrierung in Reste R³ bis R¹⁰, die für Hydroxy stehen, übergeführt werden können;
(c) Reste R³ bis R¹⁰, die für Hydroxy stehen, anschließend erneut alkyliert, acyliert oder aminoalkyliert werden können;
(d) Reste R³ bis R¹⁰, die für Amino stehen, alkyliert oder acyliert werden können;
(e) Reste R³ bis R¹⁰, die für Alkylthio stehen, zu Resten R³ bis R¹⁰, die für Alkylsulfinyl oder Alkylsulfonyl stehen, oxidiert werden können; oder auch
(f) Verbindungen der allgemeinen Formeln I oder III, in der einer oder zwei der Reste R³ bis R¹⁰ für C₁₋₄-Alkyl, C₁₋₄-Acyl, Chlor, Brom oder Nitro stehen, nach bekannten Methoden der elektrophilen aromatischen Substitution aus Verbindungen der allgemeinen Formeln I oder III, in der die betreffenden Reste R³ bis R¹⁰ für Wasserstoff stehen, hergestellt werden können.

13. Arzneimittel enthaltend Verbindungen der Formel I gemäß der Ansprüche 1 bis 11 neben üblichen Hilfs- und Zusatzstoffen.

14. Verwendung von Verbindungen der Formel I gemäß der Ansprüche 1 bis 11 zur Herstellung von Arzneimitteln zur Behandlung und/oder Prävention von Krebs, Viruserkrankungen (z.B. HIV-Infektionen), Herz- und Gefäßerkrankungen (wie z.B. Bluthochdruck, Thrombose, Herzrhythmusstörungen, Atherosklerose), bronchopulmonalen Erkrankungen, degenerativen Erkrankungen des Zentralnervensystems (z.B. Alzheimer), Entzündungskrankheiten (Z.B. Rheuma, Arthritis), Krankheiten des Immunsystems (z.B. Allergien), sowie Psoriasis oder zur Verwendung als Immunsuppressivum.

## Claims

1. Indolocarbazole imides of the general formula: 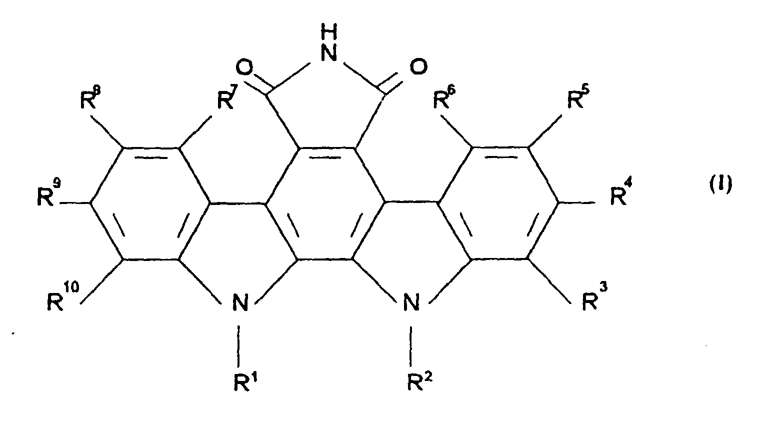 wherein
R¹ and R² are the same or different and, in each case, stand for hydrogen, methyl, ethyl, cyanomethyl, 2-cyanoethyl, 3-cyanopropyl, 3-cyano-2-propyl, 2-azidoethyl, 3-azido-propyl, 2,3-di-hydroxypropyl, 3-chloro-2-hydroxy-1-propyl, 3-aminopropyl, 3-dimethylaminopropyl, 3-trimethylammoniopropyl, 4-amino-butyl, 5-aminopentyl, epoxymethyl, 2-epoxyethyl, 3-methylaminopropyl, 3-ethylaminopropyl, 3-isopropylaminopropyl, 3-diisopropylaminopropyl, 3-methylamino-2-hydroxy-1-propyl, 3-ethylamino-2-hydroxy-1-propyl, 3-isopropylamino-2-hydroxy-1 propyl, 3-dimethylamino-2-hydroxy-1 propyl, 3-diethylamino-2-hydroxy-1-propyl, 3-diisopropylamino-2-hydroxy-1-propyl, 3-pyrrolidino-2-hydroxy-1-propyl or the radical - -(CH₂)₂CONH₂ and R³ to R¹⁰, independently of one another, stand for hydrogen, methyl, methoxy, n-propoxy, chlorine, bromine, nitro, hydroxyl or amino or two neighbouring radicals together stand for methylened ioxy radical,
with the provision that
(1.) not all of the radicals of R¹ to R¹⁰ may simultaneously be hydrogen and that
(2.) all other radicals cannot simultaneously be hydrogen when (a) R¹ is a 3-dimethylaminopropyl group, or when (b) R³ and R¹⁰ simultaneously signify chlorine, hydroxyl or methoxy, or when (c) R⁴ and/or R⁹ signify chlorine, hydroxyl or methoxy or when (d) R⁵ and/or R⁸ signify methyl, methoxy, benzyloxy, chlorine, bromine or fluorine as well as the harmless pharmacological salts thereof.

2. Indolecarbazole imides of general formula I, according to claim 1, namely:
13-(2-cyanoethyl)-6, 7, 12, 13-tetrahydro-3-methoxy-12-methyl-5, 7-dioxo-5H-indolo[2, 3-a] pyrrolo[3,4-c]-carbazole;
12-(2-cyanoethyl)-6, 7, 12, 13-tetrahydro-13-methyl-5, 7-dioxo-5H-indolo[2,3-a] pyrrolo[3,4-c]carbazole; 12-(2-cyanoethyl)-6, 7, 12, 13-tetrahydro-3-methoxy-13-methyl-5, 7-dioxo-5H-indolo[2, 3-a]pyrrolo[3,4-c]-carbazole;
12-(2-cyanoethyl)-6, 7, 12, 13-tetrahydro-3, 9-dimethoxy-13-methyl-5, 7-dioxo-5H-indolo[2,3-a]pyrrolo[3,4-c]-carbazole;
12-(3-cyano-2-propyl)-6, 7, 12, 13-tetrahydro-13-methyl-5,7-dioxo-5H-indolo[2,3-a]pyrrolo[3,4-c]carbazole;
12-Cyanomethyl-6, 7, 12, 13-tetrahydro-13 methyl 5, 7-dioxo-5H-indolo [2,3-a] pyrrolo [3,4-c]carbazole;
3-Chlor-13-(2-cyanoethyl)-6, 7, 12, 13-tetrahydro-12-methyl-5, 7-dioxo-5H-indolo [2, 3-a] pyrrolo [3, 4-c]-carbazole;
12-(3-cyanopropyl)-6, 7, 12, 13-tetrahydro-13-methyl-5,7-dioxo-5H-indolo[2, 3-a]pyrrolo [3,4-c] carbazole;
12-(3-Cyanopropyl-6, 7, 12, 13-tetrahydro-3-methoxy-13 methyl 5, 7-dioxo-5H-indolo [2,3-a] pyrrolo [3,4-c] carbazole;
12-[3-(4-methylphenylsulfphonyloxy)propyl]-6,7,12,13-tetrahydro-13-methyl-5, 7-dioxo-5H-indolo[2,3-a]pyrrolo [3, 4 c] carbazole ;
12-(3-dimethylaminopropyl)-6, 7, 12, 13-tetrahydro 13-methyl-5, 7-dioxo-5H-indolo [2, 3-a] pyrrolo [3,4-c] carbazole;
13-(3-dimethylaminopropyl)-6, 7, 12, 13-tetrahydro-3-methoxy-12-methyl-5, 7-dioxo-5H-indolo [2, 3-a] pyrrolo [3,4-c] carbazole;
12-(3-azidopropyl)-6, 7, 12, 13-tetrahydro-13-methyl-5, 7-dioxo-5H-indolo [2,3-a] pyrrolo [3,4-c] carbazole;
12-(2-azidoethyl)-6, 7, 12, 13-tetrahydro-13-methyl-5, 7, dioxo-5H-indolo [2,3-a] pyrrolo [3, 4 c] carbazole;
3-chloro-13(2-cyanoethyl)-6, 7, 12, 13 tetrahydro-9-methoxy-12-methyl 5, 7-dioxo-5H-indolo [2,3-a] pyrrolo [3,4-c] carbazole;
13-(2-cyanoethyl)-6, 7, 12, 13-tetrahydro-2, 3-dimethoxy-12-dimethoxy-12-methyl-5, 7-dioxo-5H-indolo [2, 3-a] pyrrolo [3, 4-c] carbazole;
13-(3-cyanopropyl)- 6, 7, 12, 13-tetrahydro-3-methoxy-12-methyl-5, 7-dioxo-5H-indolo [2, 3-a] pyrrolo [3,4-c] carbazole;
12-(3-cyanopropyl)-6, 7, 12, 13-tetrahydro-3, 9-dimethoxy-13-methyl-5, 7-dioxo-5H-indolo [2, 3-a] pyrrolo [3,4-c]-carbazole;
13-(2-cyanoethyl)-6, 7, 12, 13-tetrahydro-2, 3, 9-trimethoxy-12-methyl-5, 7-dioxo-5H-indolo [2,3-a] pyrrolo [3,4-c]-carbazole;
13-(2-cyanoethyl)-6, 7, 12, 13-tetrahydro-1-methoxy-12-methyl-5,7-dioxo-5H-indolo [2,3-a] pyrrolo [3,4-c]-carbazole;
13-(2-cyanoethyl)-6, 7, 12, 13-tetrahydro-2-methoxy-12-methyl-5,7-dioxo-5H-indolo [2,3-a] pyrrolo [3,4-c]-carbazole;
12-(4-azidobutyl)-6, 7, 12, 13-tetrahydro-13-methyl 5, 7-dioxo-5H-indolo [2,3-a] pyrrolo [3,4-c]-carbazole;
12-(5-azidopentyl)-6, 7, 12, 13-tetrahydro-13-methyl-5, 7-dioxo-5H-indolo [2,3-a] pyrrolo [3,4-c] carbazole;
12-(2-dimethylaminoethyl)-6, 7, 12, 13-tetrahydro-13-methyl-5, 7-dioxo-5H-indolo [2,3-a] pyrrolo [3,4-c] carbazole;
12-(3-dimethylaminopropyl)-6, 7, 12, 13-tetrahydro-3-methoxy-13 methyl 5,7-dioxo-5H-indolo [2,3-a] pyrrolo [3,4-c]-carbazole;
13-(3-dimethylaminopropyl)-6, 7, 12, 13-tetrahydro-2, 3-dimethoxy-12-methyl-5, 7-dioxo-5H-indolo [2, 3-a] pyrrolo [3,4- c]carbazole;
12-epoxymethyl-6, 7, 12, 13-tetrahydro-13-methyl-5, 7-dioxo-5H-indolo [2,3-a] pyrrolo [3,4-c] carbazole; and
(±)-12-(3-chloro-2-hydroxy-1-propyl)-6, 7, 12, 13-tetrahydro-13-methyl-5, 7-dioxo-5H-indolo [2,3-a] pyrrolo [3,4-c]carbazole.

3. Indolocarbazole imides of general formula I, according to claim 1, namely
13-(2-cyanoethyl)-6, 7, 12, 13-tetrahydro-3-hydroxy-12-methyl-5, 7-dioxo-5H-indolo [2, 3-a] pyrrolo [3,4-c]-carbazole;
12-(2-cyanoethyl)-6, 7, 12, 13-tetrahydro-3-hydroxy-13-methyl-5, 7-dioxo-5H-indolo [2,3-a] pyrrolo [3,4-c]-carbazole;
13-(2-cyanoethyl)-6, 7, 12, 13-tetrahydro-2,3-dihydroxy-12-methyl-5, 7-dioxo-5H-indolo [2,3-a] pyrrolo [3,4-c]-carbazole;
12-(3-dimethylaminopropyl)-6, 7, 12, 13-tetrahydro-3, 9-dihydroxy-5, 7-dioxo-5H-indolo [2,3-a] pyrrolo [3,4-c]-carbazole;
12-(3-dimethylaminopropyl)-6, 7, 12, 13-tetrahydro-3(9)-hydroxy-9(3)-methoxy-5, 7-dioxo-5H-indolo-[2,3-a] pyrrolo-[3,4-c] carbazole;
6, 7, 12, 13-tetrahydro-3, 9-dihydroxy-5-7-dioxo-5H-indolo [2,3-a] pyrrolo [3,4-c] carbazole;
12-(2-cyanoethyl)-6, 7, 12, 13-tetrahydro-3, 9-dihydroxy-13-methyl-5, 7-dioxo-5H-indolo[2, 3-a] pyrrolo [3,4-c] carbazole;
13-(3-dimethylaminopropyl)-6, 7, 12, 19-tetrahydro-3-hydroxy-12-methyl-5, 7-dioxo-5H-indolo [2,3-a] pyrrolo [3,4-c] carbazole; and
12-(3-diisopropylaminopropyl)-6, 7, 12, 13-tetrahydro-3-hydroxy-13-methyl-5, 7-dioxo-5H-indolo [2,3-a] pyrrolo [3,4-c] carbazole.

4. Indolocarbazole imides of general formula I, according to claim 1, namely
12-(3-dimethylaminopropyl)-6, 7, 12, 13-tetrahydro-13-methyl-5, 7-dioxo-5H indolo [2,3-a] pyrrolo [3,4-c] carbazole methiodide.

5. Indolocarbazole imides of general formula I, according to claim 1, namely
13-(3-aminopropyl)-6, 7, 12, 13-tetrahydro-3-methoxy-12-methyl-5, 7 dioxo-5H-indolo [2,3-a] pyrrolo [3,4-c]-carbazole;
13-(3-aminopropyl)-3-chloro 6, 7, 12, 13-tetrahydro-9-methoxy-12-methyl-5, 7-dioxo-5H-indolo [2,3-a] pyrrolo [3,4-c] carbazole; and
13-(3-isopropylaminopropyl)-6, 7, 12, 13-tetrahydro-13-methyl-5,7-dioxo-5H-indolo [2,3-a] pyrrolo [3,4-c]-carbazole.

6. Indolocarbazole imides of general formula I, according to claim 1, namely
12-(2-carbamoylethyl)-6, 7, 12, 13-tetrahydro-5, 7-dioxo-5H-indolo-[2,3-a] pyrrolo [3,4-c] carbazole.

7. Indolocarbazole imides of general formula I, according to claim 1, namely
(±)-12-(3-diethylamino-2-hydroxy-1-propyl)-6, 7, 12, 13-tetrahydro-5,7-dioxo-5H-indolo [2,3-a] pyrrolo-[3,4-c] carbazole.

8. Indolocarbazole imides of general formula (I), according to claim 1, namely
12-(3-dimethylaminopropyl)-6, 7, 12, 13-tetrahydro-3,9-dimethoxy-5-7 dioxo 5H indolo [2,3-a] pyrrolo [3,4-c]-carbazole;
6, 7, 12, 13-tetrahydro-3,9-dimethoxy 12-methyl-5, 7-dioxo-5H-indolo [2,3-a] pyrrolo [3,4-c] carbazole;
6, 7, 12, 13- tetrahydro-12-methyl-5, 7-dioxo-5H-indolo [2,3-a] pyrrolo [3,4-c] carbazole;
6, 7, 12, 13- tetrahydro-12-methoxy-12-methyl-5, 7-dioxo-5H-indolo [2,3-a] pyrrolo [3,4-c] carbazole;
6, 7, 12, 13- tetrahydro-12, 13-dimethyl-5, 7-dioxo-5H-indolo [2,3-a] pyrrolo [3,4-c] carbazole;
6, 7, 12, 13-tetrahydro-12-13 diethyl-5, 7-dioxo-5H-indolo [2,3-a] pyrrolo [3,4-c] carbazole;
12-(3-diisopropylaminopropyl)- 6, 7, 12, 13-tetrahydro-5,7-dioxo-5H- indolo [2,3-a] pyrrolo [3,4-c] carbazole;
12-(3-diisopropylaminopropyl) -6, 7, 12, 13-tetrahydro-13-methyl -5, 7- dioxo 5H indolo [2,3-a] pyrrolo[3,4-c] carbazole; and
6, 7, 12, 13, tetrahydro-5, 7 dioxo-3,9-di-n-propoxy-5H-indolo[2, 3-a] pyrrolo [3, 4-c] carbazole.

9. Indolocarbazole imides of general formula I, according to claim 1, namely
12-(2, 3 dihydroxy-1-propyl)-6, 7, 12, 13-tetrahydro-5, 7-dioxo-5H-indolo [2, 3-a] pyrrolo [3, 4-c] carbazole.

10. Indolocarbazole imides of general formula I, according to claim 1, namely
12-(4-aminobutyl)-6, 7, 12, 13-tetrahydro-13-methyl-5, 7-dioxo-5H-indolo [2, 3-a] pyrrolo 3,4-c] carbazole and
12-(5-aminopentyl)-6, 7, 12, 13-tetrahydro-13-methyl-5, 7-dioxo-5H-indolo [2,3-a] pyrrolo [3,4-c] carbazole,

11. Indolocarbazole imides of general formula I, according to claim 1, namely
(±)-12-(3-diethylamino-2-hydroxy-1-propyl)-6, 7, 12, 13-tetrahydro-13-methyl-5,7-dioxo-5H-indolo[2,3-a] pyrrolo [3,4-c]-carbazole;
(±)-12-(3-diethylamino-2-hydroxy-1-propyl)-6, 7, 12, 13-tetrahydro-13-methyl-5, 7-dioxo-5H-indolo [2,3-a] pyrrolo [3,4-c]carbazole;
(±)-13-(3-dimethylamino-2-hydroxy-1-propyl)-6, 7, 12, 13-tetrahydro-3-methoxy-12-methyl-5, 7-dioxo-5H-indolo [2, 3-a] pyrrolo-[ 3, 4-c]carbazole;
(±)-12-(3-dimethylamino-2-hydroxy-1-propyl)-6, 7, 12, 13-tetrahydro-3-methoxy-13-methyl-5, 7-dioxo-5H-indolo [2, 3-a] pyrrolo [3,4-c] carbozole;
(±)12-(3-diisopropylamino-2-hydroxy-1-propyl)-6, 7, 12, 13-tetrahydro-13-methyl-5,7-dioxo-5H-indolo [2,3-a] pyrrolo-[3,4-c] carbazole; and
(±)-12-(3-pyrrolidino-2-hydroxy-1-propyl)-6, 7, 12, 13-tetrahydro-13-methyl-5, 7-dioxo-5H-indolo [2,3-a] pyrrolo-[3,4-c] carbazole.

12. Process for the preparation of compounds of general formula 1, according to any one of claims 1 to 11, characterised in that
A) by oxidative cyclisation of a bis-indolylmaleimide of the general formula (II), in which R¹ to R¹⁰ have the above-given meanings and R¹³ is a hydrogen atom or a methyl radical or a protective group Z which can easily be split off, to give a compound of the general formula (I) or (III): 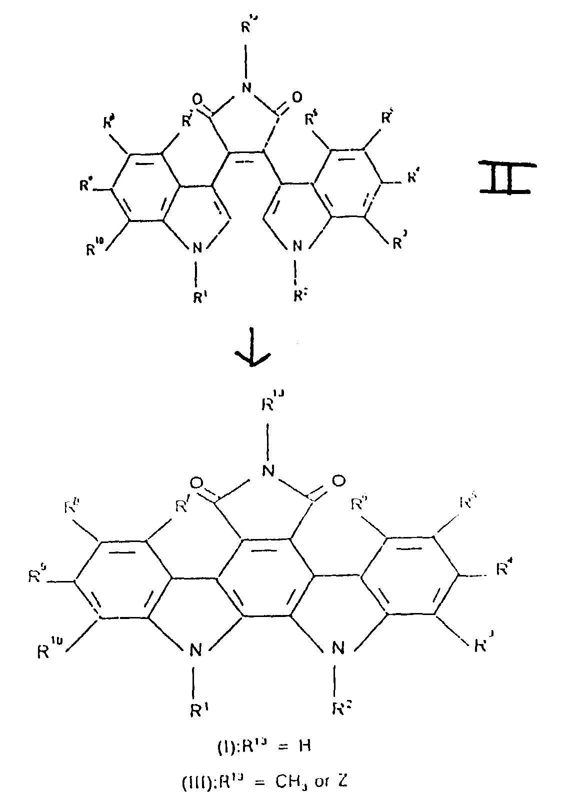 whereby
a) a compound of general formula (III), in which R¹ and/or R² is an unsubstituted, N-mono- or N,N-disubstituted 3-amino-2-hydroxy-1-propyl radical, is prepared by reaction of a compound of general formula III, in which the corresponding radicals R¹ and/or R² stand for an epoxymethyl or 2-epoxyethyl radical, with ammonia or an amine of the general formula HNRR', in which R and R' stand for a hydrogen atom or a C₁-C₄-alkyl radical or in which R and R', together with the nitrogen atom, form a heterocyclic ring containing 3 to 6 carbon atoms, which can also contain oxygen, sulphur and/or further nitrogen atoms and can be substituted by C1-C4-alkyl radicals; or
b) a compound of general formula III, in which R¹³ is a methyl radical, is converted by reaction with potassium hydroxide in water or an alcohol into the corresponding anhydride and this is converted by reaction with ammonia ammonium acetate or with hexamethyldisilazane and methanol in dimethylformamide in known manner into the corresponding unsubstituted imide of general formula I; or
c) a compound of general formula III, in which R¹³ is a protective group Z, is converted by appropriate splitting off of the protective group Z into the corresponding unsubstituted imide of general formula (I);
(B) by substitution of an indolocarbazole of the general formula Ia or IIIa: 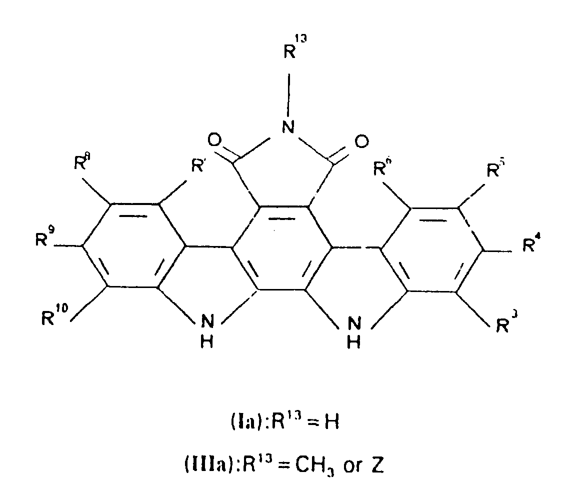 in which R¹ and R2 are hydrogen atoms, on one or both indole nitrogen atoms, for example alkylation with a compound of general formula IV
R¹⁴-X (IV)
in which R¹⁴ has one of the meanings given for R¹ and R² and X is an appropriate group which can be split off, for example chlorine, bromine, iodine or tosyl, under known reaction conditions appropriate for the alkylation of indoles or carbazoles to give a compound of general formula I or III; whereby
a) a compound of general formula I or III monosubstituted on the indole nitrogen atom, in which one of the symbols R1 and R2 stands for a hydrogen atom is converted by a further substitution on the indole nitrogen atom into a disubstituted compound of general formula I or III in which R1 and R2 have different meanings
b) a compound of general formula III is subsequently converted, as described in the case of process A, into a compound of general formula I
c) a compound of general formula (I) or (III), in which R¹ and/or R² stands for a 2-cyanoethyl or 3-cyano-2-propyl radical, is prepared by base-catalysed Michael addition of a compound of general formula (I) or (III), in which R¹and/or R² is a hydrogen atom, to an activated olefin of the general V
R¹⁵ - CH =CH-R¹⁶ (V)
in which R¹⁵ is a hydrogen atom or a methyl radical and R¹⁶ is a cyano group or R¹⁵ is a hydrogen atom and R¹⁶ is an alkoxycarbonyl radical containing up to 5 carbon atoms or -CONH₂;
d) a compound of general formula (I) or (III), in which R¹ and/or R² is an N, N-disubstituted 3-amino-2-hydroxy-1-propyl radical, is prepared by the alkylation of a compound of general formula (I) or (III), in which R¹ and/or R² is a hydrogen atom, with a 1,1-disubstituted 3-hydroxyazetidinium halide;
(f) a compound general formula (1) or (III), in which R¹ and R² together form an alkylene radical containing 2 to 4 carbon atoms, is prepared by alkylation of a compound of general formula (Ia) or (IIIa), in which R¹ and R² are hydrogen atoms, with 2 equivalents of a base and a dihaloalkane;
g) a compound of general formula (I) or (III), in which R¹ and/or R² is a methyl or ethyl radical, is prepared by alkylation in known manner with dimethyl or diethyl sulphate;
h) a compound of general formula (I) or (III), in which R¹ and/or R² is a cyano group, is prepared by the reaction of a compound of general formula (I) or (III), in which R¹ and/or R² is a hydrogen atom, with sodium hydride and phenylcyanate in dimethylformamide;
i) in the case of a compound of general formula (I) or (III), in which one or both radicals R¹ and/or R² has been introduced by reaction with an alkylation agent of general formula **IV** the introduced radical R¹ and/or R² can subsequently be modified by hydrolysis, ether cleavage, amide formation or reduction so that it has another of the meanings given R¹ and R²
j) a compound of general formula (I) or (III), in which R¹ and/or R² is a hydroxyalkyl radical, is also prepared by hydrolysis of a compound of general formula (I) or (III), in which R¹ and/or R² is a haloalkyl radical, or by ether cleavage of a compound of a general formula (I) or (III), in which R¹ and/or R² is an alkoxyalkyl radical, or by reaction of a compound of general formula (I) or (III), in which R¹ and/or R² is a hydrogen atom, with an alkylene oxide, for example propylene oxide;
I) a compound of the general formula (I) or (III), in which R¹ and/or R² is an N-unsubstituted aminoalkyl radical, is preferably prepared by catalytic hydrogenation of a compound of the general formula (I) or (III), in which R¹ and/or R² is a cyanoalkyl or azidoalkyl radical;
m) a compound of the general formula (I) or (III), in which R¹ and/or R² is an amidino alkyl radical, is prepared by known methods from a compound of the general formula (I) or (III), in which R¹ and/or R² is a cyanoalkyl or carboxyalkyl radical;
n) an N-unsubstituted aminoalkyl radical R¹ and/or R² in a compound of general formula (I) or (III) can be alkylated or acylated according to known methods and can optionally be converted according to further known methods into other functional modifications of an unsubstituted aminoalkyl radical;
o) a hydroxyalkyl radical R¹ and/or R² in a compound of general formula (I) or (III) can be functionally modified according to known methods, for example by acylation to give an acyloxyalkyl or haloalkyl radical or by reaction with an arylsulphonic acid chloride to give an arylsulphonyloxyalkyl radical;
p) an arylsulphonyloxyalkyl radical or an alkylsulphonyloxyalkyl radical R¹ and/or R² in a compound of general formula (I) or (III) can be convened in known manner by reaction with potassium cyanide to give a cyanoalkyl radical, by reaction with sodium azide to give an azidoalkyl radical, by reaction with an amine to give an aminoalkyl radical or by reaction with thiourea to give an amidinothioalkyl radical;
q) an alkylthioalkyl radical R¹ and/or R² in a compound of general formula (I) or (III) can be oxidised to give an alkylsulphinylalkyl or alkylsulphonylalkyl radical; or also
C) as described for the radicals R¹ and/or R² in a compound of general formula (I) or (III), the radicals R³ to R¹⁰ in compounds of general formula (I) or (III) can be functionally modified, whereby
a) radicals R³ to R¹⁰ which stand for nitro groups can be reduced by known methods to give radicals R³ to R¹⁰ which stand for amino groups;
b) radicals R³ to R¹⁰ which stand for methoxy radicals can be convened by ether cleavage or radical R³ to R¹⁰ which stand for benzyloxy radicals can be convened by catalytic hydrogenation into radicals R³ to R¹⁰ which stand for hydroxyl groups;
c) radicals R³ to R¹⁰ which stand for hydroxyl groups can subsequently again be alkylated, acylated or aminoalkylated;
d) radicals R³ to R¹⁰ which stand for amino groups can be alkylated or acylated;
e) radicals R³ to R¹⁰ which stand for alkylthio radicals can be oxidised to radicals R³ to R¹⁰ which strand for alkylsulphinyl or alkylsulphonyl radicals; or also
f) compounds of general formula (I) or (1ll), in which one or two of the radicals R³ to R¹⁰ stand for C1-C4-alkyl, C1,-C4-acyl, chlorine, bromine or nitro can be prepared by known methods of electrophilic aromatic substitution from compounds of general formula (I) or (III) in which the radicals R³ to R¹⁰ in question are hydrogen atoms.

13. Medicines containing at least one compound of general formula (I) according to any one of claims 1 to 11 as well as conventional adjutants and additive materials.

14. Use of compounds of the general formula I according to claims 1 to 11 for the productions of medicines for the treatment and/or prevention of cancer, virus diseases (e.g. HIV infections), heart and blood vessel diseases (e.g. high blood pressure, thromboses, heart rhythm disturbances arteriosclerosis), bronchopulmonary diseases, degenerative disease of the central nervous system (e.g. Alzheimer's disease), inflammatory diseases (e.g. rheumatism or arthritis), diseases of the immune systems (e.g. allergies), as well as psoriasis or use as an immune suppressant.

## Revendications

1. Indolocarbazol-imides de formule générale I 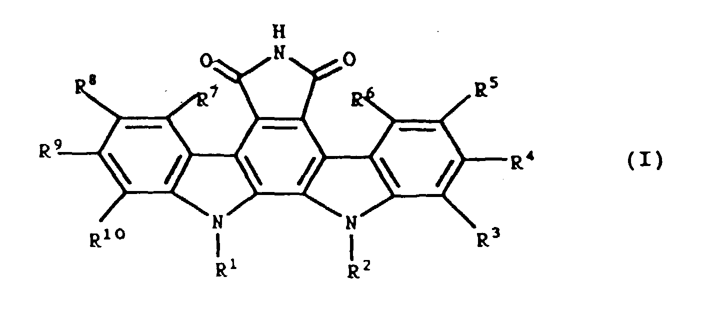 caractérisés en ce que R¹ et R² sont identiques ou différents et représentent un hydrogène, un groupe méthyle, éthyle, cyanométhyle, 2-cyanoéthyle, 3-cyanopropyle, 3-cyano-2-propyle, 2-azidoéthyle, 3-azidopropyle, 2,3-dihydroxypropyle, 3-chloro-2-hydroxy-1-propyle, 3-aminopropyle, 3-diméthylaminopropyle, 3-triméthylammoniopropyle, 4-aminobutyle, 5-aminopentyle, époxyméthyle, 2-époxyéthyle, 3-méthylaminopropyle, 3-éthylaminopropyle, 3-isopropyl-aminopropyle, 3-diisopropylaminopropyle, 3-méthylamino-2-hydroxy-1-propyle, 3-éthylamino-2-hydroxy-1-propyle, 3-isopropylamino-2-hydroxy-1-propyle, 3-diméthylamino-2-hydroxy-1-propyle, 3-diéthylamino-2-hydroxy-1-propyle, 3-diisopropylamino-2-hydroxy-1-propyle, 3-pyrrolidino-2-hydroxy-1-propyle ou un résidu -(CH₂)₂CONH₂ et R³ à R¹⁰, indépendamment les uns des autres, représentent un hydrogène, un groupe méthyle, méthoxy, n-propoxy, chlore, brome, nitro, hydroxy, amino ou bien deux résidus voisins représentent ensemble un méthylène dioxy, avec ces conditions que :
(1) tous les résidus R¹ à R¹⁰ ne sont pas simultanément des hydrogènes et que,
(2) tous les autres résidus ne sont pas en même temps des hydrogènes quand (a) R¹ est un groupe 3-diméthylaminopropyle ou lorsque (b) R³ et R¹⁰ représentent en même temps chlore, hydroxy ou méthoxy ou bien lorsque (c) R⁴ et/ou R⁹ représentent chlore, hydroxy, ou méthoxy, ou bien lorsque (d) R⁵ et/ou R⁸ représentent méthyle, méthoxy, benzyloxy, chlore, brome ou fluor, ainsi que leurs sels pharmacologiquement acceptables.

2. Indolocarbazol-imides de formule générale I selon la revendication 1, à savoir
le 13-(2-cyanoéthyl)-6,7,12,13-tétrahydro-3-méthoxy-12-méthyl-5,7-dioxo-5H-indolo[2,3-a]pyrrolo[3,4-c]carbazole ;
le 12-(2-cyanoéthyl)-6,7,12,13-tétrahydro-13-méthyl-5,7-dioxo-5H-indolo[2,3-a]pyrrolo[3,4-c]carbazole ;
le 12-(2-cyanoéthyl)-6,7,12,13-tétrahydro-3-méthoxy-13-méthyl-5,7-dioxo-5H-indolo[2,3-a]pyrrolo[3,4-c]carbazole ;
le 12-(2-cyanoéthyl)-6,7,12,13-tétrahydro-3,9-diméthoxy-13-méthyl-5,7-dioxo-5H-indolo[2,3-a]pyrrolo[3,4-c]carbazole ;
le 12-(3-cyano-2-propyl)-6,7,12,13-tétrahydro-13-méthyl-5,7-dioxo-5H-indolo[2,3-a]pyrrolo[3,4-c]carbazole ;
le 12-(cyanométhyl)-6,7,12,13-tétrahydro-13-méthyl-5,7-dioxo-5H-indolo[2,3-a]pyrrolo[3,4-c]carbazole ;
le 3-chloro-13-(2-cyanoéthyl)-6,7,12,13-tétrahydro-12-méthyl-5,7-dioxo-5H-indolo[2,3-a]pyrrolo[3,4-c]carbazole ;
le 12-(3-cyanopropyl)-6,7,12,13-tétrahydro-13-méthyl-5,7-dioxo-5H-indolo[2,3-a]pyrrolo[3,4-c]carbazole ;
le 12-(3-cyanopropyl)-6,7,12,13-tétrahydro-3-méthoxy-13-méthyl-5,7-dioxo-5H-indolo[2,3-a]pyrrolo[3,4-c]carbazole ;
le 12-[3-(4-méthylphénylsulfonyloxy)propyl]-6,7,12,13-tétrahydro-13-méthyl-5,7-dioxo-5H-indolo[2,3-a]pyrrolo[3,4-c]carbazole ;
le 12-(3-diméthylaminopropyl)-6,7,12,13-tétrahydro-13-méthyl-5,7-dioxo-5H-indolo[2,3-a]pyrrolo[3,4-c]carbazole ;
le 13-(3-diméthylaminopropyl)-6,7,12,13-tétrahydro-3-méthoxy-12-méthyl-5,7-dioxo-5H-indolo[2,3-a]pyrrolo[3,4-c]carbazole ;
le 12-(3-azidopropyl)-6,7,12,13-tétrahydro-13-méthyl-5,7-dioxo-5H-indolo[2,3-a]pyrrolo[3,4-c]carbazole ;
le 12-(2-azidoéthyl)-6,7,12,13-tétrahydro-13-méthyl-5,7-dioxo-5H-indolo[2,3-a]pyrrolo[3,4-c]carbazole ;
le 3-chloro-13-(2-cyanoéthyl)-6,7,12,13-tétrahydro-9-méthoxy-12-méthyl-5,7-dioxo-5H-indolo[2,3-a]pyrrolo[3,4-c]carbazole ;
le 13-(2-cyanoéthyl)-6,7,12,13-tétrahydro-2,3-diméthoxy-12-méthyl-5,7-dioxo-5H-indolo[2,3-a]pyrrolo[3,4-c]carbazole ;
le 13-(2-cyanoéthyl)-6,7,12,13-tétrahydro-12-méthyl-5,7-dioxo-5H-indolo[2,3-a]pyrrolo[3,4-c]carbazole ;
le 13-(3-cyanopropyl)-6,7,12,13-tétrahydro-3-méthoxy-12-méthyl-5,7-dioxo-5H-indolo[2,3-a]pyrrolo[3,4-c]carbazole ;
le 12-(3-cyanopropyl)-6,7,12,13-tétrahydro-3,9-diméthoxy-13-méthyl-5,7-dioxo-5H-indolo[2,3-a]pyrrolo[3,4-c]carbazole ;
le 13-(2-cyanoéthyl)-6,7,12,13-tétrahydro-2,3,9-triméthoxy-12-méthyl-5,7-dioxo-5H-indolo[2,3-a]pyrrolo[3,4-c]carbazole ;
le 13-(2-cyanoéthyl)-6,7,12,13-tétrahydro-1-méthoxy-12-méthyl-5,7-dioxo-5H-indolo[2,3-a]pyrrolo[3,4-]carbazole ;
le 13-(2-cyanoéthyl)-6,7,12,13-tétrahydro-2-méthoxy-12-rnéthyl-5,7-dioxo-5H-indolo[2,3-a]pyrrolo[3,4-c]carbazole ;
le 12-(4-azidobutyl)-6,7,12,13-tétrahydro-13-méthyl-5,7-dioxo-5H-indolo[2,3-a]pyrrolo[3,4-c]carbazole ;
le 12-(5-azidopentyl)-6,7,12,13-tétrahydro-13-méthyl-5,7-dioxo-5H-indolo[2,3-a]pyrrolo[3,4-c]carbazole ;
le 12-(2-diméthylaminoéthyl)-6,7,12,13-tétrahydro-13-méthyl-5,7-dîoxo-5H-indolo[2,3-a]pyrrolo[3,4-]carbazole ;
le 12-(3-diméthylaminopropyl)-6,7,12,13-tétrahydro-3-méthoxy-13-méthyl-5,7-dioxo-5H-indolo[2,3-a]pyrrolo[3,4-c]carbazole ;
le 13-(3-diméthylaminopropyl)-6,7,12,13-tétrahydro-2,3-diméthoxy-12-méthyl-5,7-dioxo-5H-indolo[2,3-a]pyrrolo[3,4-c]carbazole ;
le 12-époxyméthyl-6,7,12,13-tétrahydro-13-méthyl-5,7-dioxo-5H-indolo[2,3-a]pyrrolo[3,4-c]carbazole ;
le (±)-12-(3-chloro-2-hydroxy-1-propyl)-6,7,12,13-tétrahydro-13-méthyl-5,7-dioxo-5H-indolo[2,3-a]pyrrolo[3,4-c]carbazole.

3. Indolocarbazol-imides de formule générale I, selon la revendication 1, à savoir
le 13-(2-cyanoéthyl)-6,7,12,13-tétrahydro-3-hydroxy-12-méthyl-5,7-dioxo-5H-indolo[2,3-a]pyrrolo[3,4-c]carbazole ;
le 12-(2-cyanoéthyl)-6,7,12,13-tétrahydro-3-hydroxy-13-méthyl-5,7-dioxo-5H-indolo[2,3-a]pyrrolo[3,4-c]carbazole ;
le 13-(2-cyanoéthyl)-6,7,12,13-tétrahydro-2,3-dihydroxy-12-méthyl-5,7-dioxo-5H-indolo[2,3-a]pyrrolo[3,4-c]carbazole ;
le 12-(3-diméthylaminopropyl)-6,7,12,13-tétrahydro-3,9-dihydroxy-5,7-dioxo-5H-indolo[2,3-a]pyrrolo[3,4-c]carbazole ;
le 12-(3-diméthylaminopropyl)-6,7,12,13-tétrahydro-3(9)-hydroxy-9(3)-méthoxy-5,7-dioxo-5H-indolo[2,3-a]pyrrolo[3,4-c]carbazole ;
le 6,7,12,13-tétrahydro-3,9-dihydroxy-5,7-dioxo-5H-indolo[2,3-a]pyrrolo[3,4-c]carbazole ;
le 12-(2-cyanoéthyl)-6,7,12,13-tétrahydro-3,9-dihydroxy-13-méthyl-5,7-dioxo-5H-indolo[2,3-a]pyrrolo[3,4-c]carbazole ;
le 13-(3-diméthylaminopropyl)-6,7,12,13-tétrahydro-3-hydroxy-12-méthyl-5,7-dioxo-5H-indolo[2,3-a]pyrrolo[3,4-c]carbazole ;
le 12-(3-diisopropylaminopropyl)-6,7,12,13-tétrahydro-3-hydroxy-13-méthyl-5,7-dioxo-5H-indolo[2,3-a]pyrrolo[3,4-c]carbazole.

4. Indolocarbazol-imides de formule générale I, selon la revendication 1, à savoir :
le 12-(3-diméthylaminopropyl)-6,7,12,13-tétrahydro-13-méthyl-5,7-dioxo-5H-indolo[2,3-a]pyrrolo[3,4-c]carbazol méthoiodure.

5. Indolocarbazol imides de formule générale I, selon la revendication 1, à savoir :
le 13-(3-aminopropyl)-6,7,12,13-tétrahydro-3-méthoxy-12-méthyl-5,7-dioxo-5H-indolo[2,3-a]pyrrolo[3,4-c]carbazole ;
le 13-(3-aminopropyl)-3-chloro-6,7,12,13-tétrahydro-9-méthoxy-12-méthyl-5,7-dioxo-5H-indolo[2,3-a]pyrrolo[3,4-c]carbazole ;
le 13-(3-isopropylaminopropyl)-6,7,12,13-tétrahydro-13-méthyl-5,7-dioxo-5H-indolo[2,3-a]pyrrolo[3,4-c]carbazole.

6. Indolocarbazol-imide de formule générale I, selon la revendication 1, à savoir :
le 12-(2-carbamoyléthyl)-6,7,12,13-tétrahydro-5,7-dioxo-5H-indolo[2,3-a]pyrrolo[3,4-c]carbazole.

7. Indolocarbazol-imide de formule générale I, selon la revendication 1, à savoir :
le (±)-12-(3-diéthylamino-2-hydroxy-1-propyl)-6,7,12,13-tétrahydro-5,7-dioxo-5H-indolo[2,3-a]pyrrolo[3,4-c]carbazole.

8. Indolocarbazol-imides de formule générale I, selon la revendication 1, à savoir :
le 12-(3-diméthylaminopropyl)-6,7,12,13-tétrahydro-3,9-diméthoxy-5,7-dioxo-5H-indolo[2,3-a]pyrrolo[3,4-c]carbazole ;
le 6,7,12,13-tétrahydro-3,9-diméthoxy-12-méthyl-5,7-dioxo-5H-indolo[2,3-a]pyrrolo[3,4-c]carbazole ;
le 6,7,12,13-tétrahydro-12-méthyl-5,7-dioxo-5H-indolo[2,3-a]pyrrolo[3,4-c]carbazole ;
le 6,7,12,13-tétrahydro-3-méthoxy-12-méthyl-5,7-dioxo-5H-indolo[2,3-a]pyrrolo[3,4-c]carbazole ;
le 6,7,12,13-tétrahydro-12,13-diméthyl-5,7-dioxo-5H-indolo[2,3-a]pyrrolo[3,4-c]carbazole ;
le 6,7,12,13-tétrahydro-12,13-diéthyl-5,7-dioxo-5H-indolo[2,3-a]pyrrolo[3,4-c]carbazole ;
le 12-(3-diisopropylaminopropyl)-6,7,12,13-tétrahydro-5,7-dioxo-5H-indolo[2,3-a]pyrrolo[3,4-c]carbazole ;
le 12-(3-diisopropylaminopropyl)-6,7,12,13-tétrahydro-13-méthyl-5,7-dioxo-5H-indolo[2,3-a]pyrrolo[3,4-c]carbazole ; et
le 6,7,12,13-tétrahydro-5,7-dioxo-3,9-di-n-propoxy-5H-indolo[2,3-a]pyrrolo[3,4-c]carbazole.

9. Indolocarbazol-imide de formule générale I, selon la revendication 1, à savoir :
le 12-(2,3-dihydroxy-1-propyl)-6,7,12,13-tétrahydro-5,7-dioxo-5H-indolo[2,3-a]pyrrolo[3,4-c]carbazole.

10. Indolocarbazol-imides de formule générale I, selon la revendication 1, à savoir :
le 12-(4-aminobutyl)-6,7,12,13-tétrahydro-13-méthyl-5,7-dioxo-5H-indolo[2,3-a]pyrrolo[3,4-c]carbazole et
le 12-(5-aminopentyl)-6,7,12,13-tétrahydro-13-méthyl-5,7-dioxo-5H-indolo[2,3-a]pyrrolo[3,4-c]carbazole.

11. Indolocarbazol-imides de formule générale I, selon la revendication 1, à savoir :
le (±)-12-(3-diméthylamino-2-hydroxy-1-propyl)-6,7,12,13-tétrahydro-13-méthyl-5,7-dioxo-5H-indolo[2,3-a]pyrrolo[3,4-c]carbazole ;
le (±)-12-(3-diéthylamino-2-hydroxy-1-propyl)-6,7,12,13-tétrahydro-13-méthyl-5,7-dioxo-5H-indolo[2,3-a]pyrrolo[3,4-c]carbazole ;
le (±)-13-(3-diméthylamino-2-hydroxy-1-propyl)-6,7,12,13-tétrahydro-3-méthoxy-12-méthyl-5,7-dioxo-5H-indolo[2,3-a]pyrrolo[3,4-c]carbazole ;
le (±)-12-(3-diméthylamino-2-hydroxy-1-propyl)-6,7,12,13-tétrahydro-3-méthoxy-13-méthyl-5,7-dioxo-5H-indolo[2,3-a]pyrrolo[3,4-c]carbazole ;
le (±)-12-(3-diisopropylamino-2-hydroxy-1-propyl)-6,7,12,13-tétrahydro-13-méthyl-5,7-dioxo-5H-indolo[2,3-a]pyrrolo[3,4-c]carbazole ;
le (±)-12-(3-pyrrolidino-2-hydroxy-1-propyl)-6,7,12,13-tétrahydro-13-méthyl-5,7-dioxo-5H-indolo[2,3-a]pyrrolo[3,4-c]carbazole.

12. Un procédé de préparation de composés I selon les revendications 1 à 11, caractérisé en ce que
A) par cyclisation oxydativé de bisindolylmaléimides II, dans lesquels R¹ à R¹⁰ ont la signification donnée ci-dessus et R¹³ représente un hydrogène, un méthyle ou un groupe protecteur Z facilement éliminable, ceux-ci sont transformés en composés I ou bien en composés III, 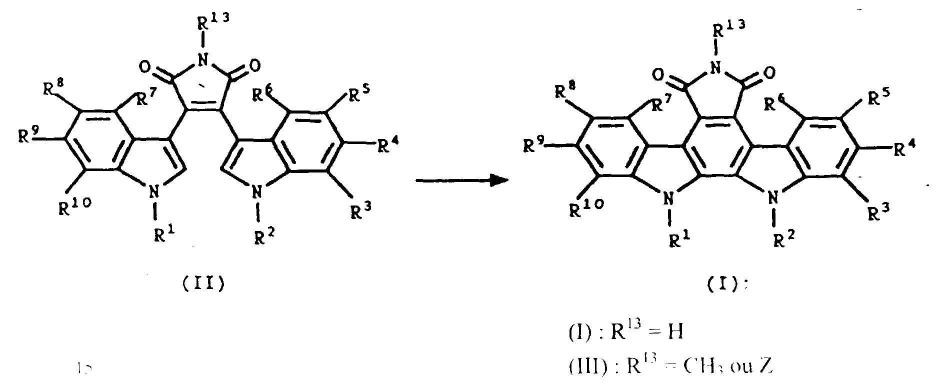 dans lequel :
(a) on prépare les composés III, dans lesquels R¹ et/ou R² représentent un résidu non substitué, N-mono- ou N,N-disubstitué 3-amino-2-hydroxy-1-propyle, par réaction de composés III, dont les résidus R¹ et/ou R² correspondants représentent un groupe époxyméthyle ou 2-époxyéthyle, avec l'ammoniac ou des amines de formule générale HNRR', où R et R' représentent un hydrogène ou un groupe alkyle en C₁ à C₄ ou dans laquelle R et R' forment ensemble avec l'atome d'azote un cycle hétérocyclique à 3 à 6 atomes de carbone, pouvant comporter également des atomes d'oxygène, de soufre et/ou d'autres atomes d'azote et pouvant être substitués par des restes alkyle en C₁ à C₄ ;
(b) on transforme les composés III, dans lesquels R¹³ représente un groupe méthyle, par réaction avec l'hydroxyde de potassium dans l'eau ou dans des alcools en les anhydrides correspondants et ceux-ci par réaction avec l'ammoniac, l'acétate d'ammonium ou avec hexaméthyldisilazane et le méthanol dans le diméthylformamide d'une manière connue en soi en les imides I non substitués correspondants, ou bien
(c) on transforme les composés III, dans lesquels R¹³ représente un groupe protecteur Z, par élimination convenable du groupe protecteur Z, en les imides I non substitutés correspondants ;
B) on prépare les composés I ou III par substitution de indolocarbazoles de formule générale la ou IIIa, 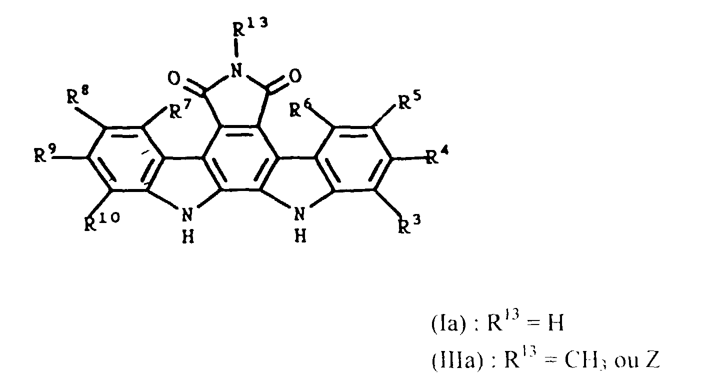 dans lesquelles R¹ et R² représentent de l'hydrogène, sur l'un ou les deux atomes d'azote indoliques, par exemple par alkylation à l'aide d'un composé IV.
R¹⁴-X (IV)
où R¹⁴ a la même signification que celle qui a été indiquée pour R¹ et R² et X est un groupe partant convenable, comme par exemple le chlore, le brome, l'iode ou le tosyle, dans des conditions réactionnelles connues, convenables pour l'alkylation des indoles, ou des carbazoles ; où.
(a) les composés de formule générale I ou III mono-substitués au niveau de l'atome d'azote indolique dans lesquels un des restes R¹ ou R² représente l'hydrogène, sont transformés par substitution additionnelle en composés de formule générale I ou III disubstitués au niveau de l'atome d'azote indolique dans lesquels R¹ et R² ont des significations différentes ;
(b) les composés de formule générale III sont ensuite transformés en composés de formule générale I selon le procédé A déjà décrit ;
(c) on prépare les composés de formule générale I ou III, dans lesquels R¹ et/ou R² représentent un groupe 2-cyanoéthyle ou bien 3-cyano-2-propyle, par addition de Michael catalysé par des bases de composés de formule générale I ou III, dans lesquels R¹ et/ou R² représentent un hydrogène, à des oléfines activées de formule générale V,
R⁵-CH=CH-R¹⁶ (V)
dans laquelle R¹⁵ représente un hydrogène ou un méthyle et R¹⁶ représente un groupe cyano, ou bien R¹⁵ représente un hydrogène et R¹⁶ représente un reste alcoxycarbonyle comportant jusqu'à 5 atomes de carbone ou un CONH₂ ;
(d) on prépare les composés de formule générale I ou III, dans lesquels R¹ et/ou R² représentent un groupe 3-amino-2-hydroxy-1-propyle N,N-disubstitué par alkylation de composés de formule générale I ou III, dans lesquels R¹ et/ou R² représentent un hydrogène, avec des halogénures de 3-hydroxy-azétidinium 1,1-disubstitués ;
(f) on prépare des composés de formule générale I ou III, dans lesquels R¹ et R² forment ensemble un groupe alkylène comportant 2 à 4 atomes de carbone, par alkylation de composés de formule générale Ia ou IIIe, où R¹ et R² représentent un hydrogène, avec deux équivalents d'une base et d'un dihalogéno-alcane ;
(g) on prépare des composés de formule générale I ou III, dans lesquels R¹ et/ou R² représentent un résidu méthyle ou éthyle, aussi par alkylation à l'aide de diméthyl- ou de diéthylsulfate d'une manière connue ;
(h) on prépare des composés de formule générale I ou III, dans lesquels R¹ et/ou R² représentent un groupe cyano, par réaction de composés de formule générale I ou III, où R¹ et/ou R² représentent un atome d'hydrogène, avec l'hydrure de sodium et le phénylcyanate dans le diméthylformamide ;
(i) dans les composés de formule générale I ou III, dans lesquels l'un ou les deux résidus R¹ et/ou R² ont été introduits par réaction avec un agent d'alkylation de formule générale IV, les résidus R¹ et/ou R² introduits peuvent ensuite être modifiés par hydrolyse, coupure de l'éther, formation d'amide ou réduction de façon à obtenir un composé dans lequel R¹ et R² ont d'autres significations indiquées ;
(j) on prépare des composés de formule générale I ou III, dans lesquels R¹ et/ou R² représentent un groupe hydroxyalkyle, également par hydrolyse de composés de formule générale I ou III, dans lesquels R¹ et/ou R² représentent un reste halogénoalkyle, ou bien par coupure d'éther de composés de formule générale I ou III, dans lesquels R¹ et/ou R² représentent un groupe alkoxyalkyle ou par réaction de composés de formule générale I ou III, dans lesquels R¹ et/ou R² représentent un hydrogène, avec un alkylèneoxyde, par exemple l'oxyde de propylène ;
(l) on prépare des composés de formule générale I ou III, dans lesquels R¹ et/ou R² représentent un reste aminoalkyle non substitué sur l'azote, de préférence par hydrogénation catalytique de composés de formule générale I ou III, dans lesquels R¹ et/ou R² représentent un résidu cyanoalkyle ou azidoalkyle :
(m) on prépare les composés de formule générale I ou III, dans lesquels R¹ et/ou R² représentent un reste amidinoalkyle, également par des méthodes connues à partir de composés de formule générale I ou III, dans lesquels R¹ et/ou R² représentent un résidu cyanoalkyle ou un groupe carboxyalkyle ;
(n) les groupes aminoalkyle R¹ et/ou R² non substitués sur l'azote peuvent être alkylés ou alcylés en composés de formule générale I ou III, par des méthodes connues, et le cas échéant, par d'autres méthodes connues également peuvent être transformés en d'autres variantes fonctionnalisées d'un groupe aminoalkyle non substitués ;
(o) les groupes hydroxyalkyles R¹ et/ou R² des composés de formule générale I ou III peuvent être modifiés fonctionnellement par des méthodes connues, par exemple par acylation en groupes acyloxyalkyles, en groupes halogénoalkyles ou par transformation avec des chlorures d'acides arylsulfonyle en groupes arylsulfonyloxyalkyles ;
(p) les groupes arylsulfonyloxyalkyles et alkylsulfonyloxyalkyles R¹ et/ou R² dans les composés de formule générale I ou III peuvent être transformés d'une manière connue par réaction avec le cyanure de potassium en des groupes cyanoalkyles, par réaction avec l'azide de sodium en des groupes azidoalkyles, par réaction avec des amines en des groupes aminoalkyles ou par réaction avec de la thiourée en des groupes amidinothioalkyles ;
(q) les groupes alkylthioalkyles R¹ et/ou R² dans les composés de formule générale I ou III peuvent être oxydés en groupes alkylsulfinylalkyles ou en groupes alkylsulfonylalkyles ; ou bien également
(C) on soumet à une modification fonctionnelle, comme décrit pour les résidus R¹ et/ou R² des composés de formule générale I ou III, les résidus R³ à R¹⁰ des composés de formule générale I ou III ;
(a) les résidus R³ à R¹⁰ qui représentent des groupes nitro peuvent être réduits par les méthodes connues en résidus R³ à R¹⁰ représentant des groupes amino ;
(b) les résidus R³ à R¹⁰, qui représentent des groupes méthoxy, par coupure d'éther, ou bien les résidus R³ à R¹⁰, qui représentent des groupes benzyloxy par hydrogénation catalytique, peuvent être transformés en résidus R³ à R¹⁰, qui représentent des groupes hydroxy ;
(c) les résidus R³ à R¹⁰, qui représentent des groupes hydroxy, peuvent être ensuite de nouveau alkylés, acylés ou aminoalkylés ;
(d) les résidus R³ à R¹⁰, qui représentent des groupes amino, peuvent être alkylés ou acylés ;
(e) les résidus R³ à R¹⁰, qui représentent des groupes alkylthio peuvent être oxydés en des résidus R³ à R¹⁰ qui représentent des groupes alkylsulfinyle ou alkylsulfonyle ; ou bien également
(f) les composés de formule générale I ou III, dans lesquels un ou deux des résidus R³ à R¹⁰ représentent un alkyle en C₁ à C₄, acyle en C₁ à C₄, chlore, brome ou nitro, peuvent être obtenus par des méthodes connues de substitution aromatique électrophile à partir de composés de formule générale I ou III, dans lesquels les résidus R³ à R¹⁰ correspondants représentent des hydrogènes.

13. Médicaments renfermant des composés de formule I selon une quelconque des revendications 1 à 11 en association avec les véhicules et adjuvants habituels.

14. Utilisation de composés de formule I selon l'une quelconque des revendications 1 à 11 pour la préparation d'un médicament pour le traitement et/ou la prévention du cancer des maladies virales (par exemple infection du SIDA), maladies du coeur et de la circulation (comme par exemple, hypertension artérielle, thrombose, troubles du rythme cardiaque, athérosclérose), maladies bronchopulmonaires, maladies dégénératives du système nerveux central (par exemple maladie d'Alzheimer), maladies inflammatoires (par exemple rhumatisme, arthrite), maladies du système immunitaire (par exemple allergies) ainsi que psoriasis ou pour servir comme immunodépresseur.
